# EUROPEAN PATENT SPECIFICATION

(11) **EP 1 375 510 B1**
(45) Date of publication and mention of the grant of the patent: **04.06.2008**
(21) Application number: 02077574.8
(22) Date of filing: 28.06.2002
(51) Int. Cl.: C07K 14/00, A61K 38/00

(54) **Modification of collagenous materials and medical treatment, diagnosis and monitoring of fibrotic conditions**
Modifikation von Kollagen Material und Medizinische Behandlung, Diagnose, und Monitoring von Fibrotischen Konditionen
Modification du colagene et traitement médical, diagnose, et surveillance des conditions fibrotiques

(43) Date of publication of application: 02.01.2004
(73) Proprietor: Nederlandse Organisatie voor Toegepast-Natuurwetenschappelijk Onderzoek TNO, 2628 VK Delft (NL)
(72) Inventor: te Koppele, Johannes Maria, 2353 WP Leiderdorp (NL); Bank, Ruud Antonius, 2132 EL Hoofddorp (NL)
(74) Representative: Prins, Adrianus Willem

(56) References cited:
- RUOTSALAINEN H ET AL: "Complete genomic structure of mouse lysyl hydroxylase 2 and lysyl hydroxylase 3/collagen glucosyltransferase." MATRIX BIOLOGY: JOURNAL OF THE INTERNATIONAL SOCIETY FOR MATRIX BIOLOGY. GERMANY APR 2001, vol. 20, no. 2, April 2001 (2001-04), pages 137-146, XP001117877 ISSN: 0945-053X
- BANK RUUD A ET AL: "Defective collagen crosslinking in bone, but not in ligament or cartilage, in Bruck syndrome: Indications for a bone-specific telopeptide lysyl hydroxylase on chromosome 17." PROCEEDINGS OF THE NATIONAL ACADEMY OF SCIENCES OF THE UNITED STATES, vol. 96, no. 3, 2 February 1999 (1999-02-02), pages 1054-1058, XP001117878 Feb. 2, 1999 ISSN: 0027-8424
- HEATHER N YEOWELL ET AL: "Characterization of a partial cDNA for lysyl hydroxylase from human skin fibroblasts;lysyl hydroxylase mRNAs are regulated differently by minoxidil derivatives and hydralazine" JOURNAL OF INVESTIGATIVE DERMATOLOGY, NEW YORK, NY, US, vol. 99, 1992, pages 864-869, XP002902024 ISSN: 0022-202X
- FRIESS W: "COLLAGEN - BIOMATERIAL FOR DRUG DELIVERY" EUROPEAN JOURNAL OF PHARMACEUTICS AND BIOPHARMACEUTICS, ELSEVIER SCIENCE PUBLISHERS B.V., AMSTERDAM, NL, vol. 45, no. 2, 1 March 1998 (1998-03-01), pages 113-136, XP000738297 ISSN: 0939-6411
- UZAWA KATSUHIRO ET AL: "Differential expression of human lysyl hydroxylase genes, lysine hydroxylation, and cross-linking of type I collagen during osteoblastic differentiation in vitro." JOURNAL OF BONE AND MINERAL RESEARCH, vol. 14, no. 8, August 1999 (1999-08), pages 1272-1280, XP001118669 ISSN: 0884-0431

### SEQUENCE LISTING

<110> Nederlandse Organisatie voor toegepast natuurwetenschappelijk Onderzoek TNO
<120> Modification of collagenous materials and medical treatment, diagnosis monitoring of fibrotic conditions
<130> P60187EP00
<140> 02077574.8
   <141> 2002-06-28
<160> 59
<170> PatentIn Ver. 2.1
<210> 1
   <211> 8
   <212> PRT
   <213> Artificial Sequence
<220>
   <223> Description of Artificial sequence: part of PLOD2 in which the missense mutation found in Bruck syndrome family PM is present
<220>
   <221> SITE
   <222> (1)..(8)
<400> 1
<210> 2
   <211> 9
   <212> PRT
   <213> Artificial Sequence
<220>
   <223> Description of Artificial sequence: helical sequence
<220>
   <221> SITE
   <222> (1)..(9)
<400> 2
   Ile Lys Gly Ile Lys Gly Ile Lys Gly
   1 5
<210> 3
   <211> 12
   <212> PRT
   <213> Artificial sequence
<220>
   <223> Description of Artificial Sequence: helical sequence
<220>
   <221> SITE
   <222> (1)..(12)
<400> 3
   Ala Arg Gly Ile Lys Gly Ile Arg Gly Phe Ser Gly
   1 5 10
<210> 4
   <211> 20
   <212> DNA
   <213> Artificial sequence
<220>
   <223> Description of Artificial sequence: forward primer promoter
<220>
   <221> misc_feature
   <222> (1)..(20)
<400> 4
   ctcccaaagc taagtgcagg 20
<210> 5
   <211> 20
   <212> DNA
   <213> Artificial sequence
<220>
   <223> Description of Artificial sequence: forward primer exon 1
<220>
   <221> misc_feature
   <222> (1)..(20)
<400> 5
   gtctctgcgt tctcgcgaga 20
<210> 6
   <211> 23
   <212> DNA
   <213> Artificial Sequence
<220>
   <223> Description of Artificial sequence: forward primer exon 2
<220>
   <221> misc_feature
   <222> (1)..(23)
<400> 6
   tgaggtctca attactgtag tga 23
<210> 7
   <211> 23
   <212> DNA
   <213> Artificial sequence
<220>
   <223> Description of Artificial Sequence: forward primer exon 3
<220>
   <221> misc_feature
   <222> (1)..(23)
<400> 7
   gtactgttca agttgatgat gtc 23
<210> 8
   <211> 24
   <212> DNA
   <213> Artificial Sequence
<220>
   <223> Description of Artificial Sequence: forward primer exon 4
<220>
   <221> misc_feature
   <222> (1)..(24)
<400> 8
   atggtttatg tgcctagatt ctga 24
<210> 9
   <211> 21
   <212> DNA
   <213> Artificial Sequence
<220>
   <223> Description of Artificial Sequence: forward primer exon 5
<220>
   <221> misc_feature
   <222> (1)..(21)
<400> 9
   ttctttcatg gtgagctgtg a 21
<210> 10
   <211> 22
   <212> DNA
   <213> Artificial Sequence
<220>
   <223> Description of Artificial Sequence: forward primer exon 6
<220>
   <221> misc_feature
   <222> (1)..(22)
<400> 10
   gcaactatcg cagtttctac ct 22
<210> 11
   <211> 22
   <212> DNA
   <213> Artificial sequence
<220>
   <223> Description of Artificial Sequence: forward primer exon 7
<220>
   <221> misc_feature
   <222> (1)..(22)
<400> 11
   cacatacaca cacagacaca cg 22
<210> 12
   <211> 23
   <212> DNA
   <213> Artificial Sequence
<220>
   <223> Description of Artificial sequence: forward primer exon 8
<220>
   <221> misc_feature
   <222> (1)..(23)
<400> 12
   taaaggaata tacctgctgc aga 23
<210> 13
   <211> 23
   <212> DNA
   <213> Artificial sequence
<220>
   <223> Description of Artificial Sequence: forward primer exon 9
<220>
   <221> misc_feature
   <222> (1)..(23)
<400> 13
   tttcaagtgt tagagaactg cca 23
<210> 14
   <211> 23
   <212> DNA
   <213> Artificial Sequence
<220>
   <223> Description of Artificial sequence: forward primer exon 10
<220>
   <221> misc_feature
   <222> (1)..(23)
<400> 14
   tctaagattt ctaggctaca ggc 23
<210> 15
   <211> 23
   <212> DNA
   <213> Artificial Sequence
<220>
   <223> Description of Artificial sequence: forward primer exon 11
<220>
   <221> misc_feature
   <222> (1)..(23)
<400> 15
   cagaaaagta tgctagagaa cca 23
<210> 16
   <211> 23
   <212> DNA
   <213> Artificial sequence
<220>
   <223> Description of Artificial Sequence: forward primer exon 12
<220>
   <221> misc_feature
   <222> (1)..(23)
<400> 16
   caggtttgtt gaatgagctt tct 23
<210> 17
   <211> 21
   <212> DNA
   <213> Artificial sequence
<220>
   <223> Description of Artificial Sequence: forward primer exon 13
<220>
   <221> misc_feature
   <222> (1)..(21)
<400> 17
   ggggcagtgg tttatctcct a 21
<210> 18
   <211> 21
   <212> DNA
   <213> Artificial Sequence
<220>
   <223> Description of Artificial Sequence: forward primer exon 13a
<220>
   <221> misc_feature
   <222> (1)..(21)
<400> 18
   agaatacctg agagagcggg t 21
<210> 19
   <211> 22
   <212> DNA
   <213> Artificial Sequence
<220>
   <223> Description of Artificial sequence: forward primer exon 14
<220>
   <221> misc_feature
   <222> (1)..(22)
<400> 19
   cagttgagtg tcagtgctat ct 22
<210> 20
   <211> 23
   <212> DNA
   <213> Artificial Sequence
<220>
   <223> Description of Artificial Sequence: forward primer exon 15
<220>
   <221> misc_feature
   <222> (1)..(23)
<400> 20
   ataagcatat tcagaaccag gca 23
<210> 21
   <211> 22
   <212> DNA
   <213> Artificial sequence
<220>
   <223> Description of Artificial Sequence: forward primer exon 16
<220>
   <221> misc_feature
   <222> (1)..(22)
<400> 21
   tcatcaattc tgaggtgcac ca 22
<210> 22
   <211> 23
   <212> DNA
   <213> Artificial Sequence
<220>
   <223> Description of Artificial Sequence: forward primer exon 17 / 18 / 19
<220>
   <221> misc_feature
   <222> (1)..(23)
<400> 22
   agcagatgat ataccacatt gga 23
<210> 23
   <211> 19
   <212> DNA
   <213> Artificial sequence
<220>
   <223> Description of Artificial Sequence: reverse primer promoter
<220>
   <221> misc_feature
   <222> (1)..(19)
<400> 23
   agacagggat tccaggggt 19
<210> 24
   <211> 22
   <212> DNA
   <213> Artificial sequence
<220>
   <223> Description of Artificial Sequence: reverse primer exon 1
<220>
   <221> misc_feature
   <222> (1)..(22)
<400> 24
   aagggctgtt ggatgaatga ac 22
<210> 25
   <211> 23
   <212> DNA
   <213> Artificial sequence
<220>
   <223> Description of Artificial Sequence: reverse primer exon 2
<220>
   <221> misc_feature
   <222> (1)..(23)
<400> 25
   cttccttgtg aggattacag att 23
<210> 26
   <211> 21
   <212> DNA
   <213> Artificial Sequence
<220>
   <223> Description of Artificial Sequence: reverse primer exon 3
<220>
   <221> misc_feature
   <222> (1)..(21)
<400> 26
   gccaccgtgc ccaaccatat t 21
<210> 27
   <211> 23
   <212> DNA
   <213> Artificial Sequence
<220>
   <223> Description of Artificial Sequence: reverse primer exon 4
<220>
   <221> misc_feature
   <222> (1)..(23)
<400> 27
   ggaacaccaa ctcacataat aca 23
<210> 28
   <211> 20
   <212> DNA
   <213> Artificial Sequence
<220>
   <223> Description of Artificial Sequence: reverse primer exon 5
<220>
   <221> misc_feature
   <222> (1)..(20)
<400> 28
   tgatatccag ccaggtgaca 20
<210> 29
   <211> 24
   <212> DNA
   <213> Artificial sequence
<220>
   <223> Description of Artificial Sequence: reverse primer exon 6
<220>
   <221> misc_feature
   <222> (1)..(24)
<400> 29
   ccaaatggac ataacaaagg aaag 24
<210> 30
   <211> 22
   <212> DNA
   <213> Artificial Sequence
<220>
   <223> Description of Artificial Sequence: reverse primer exon 7
<220>
   <221> misc_feature
   <222> (1)..(22)
<400> 30
   aaaggctatc actctgctga gg 22
<210> 31
   <211> 23
   <212> DNA
   <213> Artificial Sequence
<220>
   <223> Description of Artificial Sequence: reverse primer exon 8
<220>
   <221> misc_feature
   <222> (1)..(23)
<400> 31
   attccactta catctactgc aga 23
<210> 32
   <211> 23
   <212> DNA
   <213> Artificial Sequence
<220>
   <223> Description of Artificial Sequence: reverse primer exon 9
<220>
   <221> misc_feature
   <222> (1)..(23)
<400> 32
   ccactgaact taacccaatg aat 23
<210> 33
   <211> 22
   <212> DNA
   <213> Artificial Sequence
<220>
   <223> Description of Artificial sequence: reverse primer exon 10
<220>
   <221> misc_feature
   <222> (1)..(22)
<400> 33
   gttggctact gcatacgcaa ac 22
<210> 34
   <211> 23
   <212> DNA
   <213> Artificial sequence
<220>
   <223> Description of Artificial Sequence: reverse primer exon 11
<220>
   <221> misc_feature
   <222> (1)..(23)
<400> 34
   gtagaacata actaagttcc ctc 23
<210> 35
   <211> 21
   <212> DNA
   <213> Artificial sequence
<220>
   <223> Description of Artificial Sequence: reverse primer exon 12
<220>
   <221> misc_feature
   <222> (1)..(21)
<400> 35
   aggattccaa gtggtcttgg g 21
<210> 36
   <211> 21
   <212> DNA
   <213> Artificial Sequence
<220>
   <223> Description of Artificial Sequence: reverse primer exon 13
<220>
   <221> misc_feature
   <222> (1)..(21)
<400> 36
   cacagtgaca caccaactgg t 21
<210> 37
   <211> 22
   <212> DNA
   <213> Artificial sequence
<220>
   <223> Description of Artificial sequence: reverse primer exon 13a
<220>
   <221> misc_feature
   <222> (1)..(22)
<400> 37
   acgcaaacac acagatgact ga 22
<210> 38
   <211> 22
   <212> DNA
   <213> Artificial Sequence
<220>
   <223> Description of Artificial sequence: reverse primer exon 14
<220>
   <221> misc_feature
   <222> (1)..(22)
<400> 38
   ctggtgtgag acagtatctc at 22
<210> 39
   <211> 22
   <212> DNA
   <213> Artificial sequence
<220>
   <223> Description of Artificial Sequence: reverse primer exon 15
<220>
   <221> misc_feature
   <222> (1)..(22)
<400> 39
   ctccactttc acatcttctg tg 22
<210> 40
   <211> 20
   <212> DNA
   <213> Artificial sequence
<220>
   <223> Description of Artificial sequence: reverse primer exon 16
<220>
   <221> misc_feature
   <222> (1)..(20)
<400> 40
   agaaacccgc ccaaactaat 20
<210> 41
   <211> 22
   <212> DNA
   <213> Artificial sequence
<220>
   <223> Description of Artificial sequence: reverse primer exon 17 / 18 / 19
<220>
   <221> misc_feature
   <222> (1)..(22)
<400> 41
   gttcatgcca gtcattcatc ca 22
<210> 42
   <211> 20
   <212> DNA
   <213> Artificial sequence
<220>
   <223> Description of Artificial Sequence: sequence primer for exon 18
<220>
   <221> misc_feature
   <222> (1)..(20)
<400> 42
   ggtctttgca ggctattata 20
<210> 43
   <211> 19
   <212> DNA
   <213> Artificial sequence
<220>
   <223> Description of Artificial Sequence: sequence primer for exon 19
<220>
   <221> misc_feature
   <222> (1)..(19)
<400> 43
   gctcaaatga cataatttg 19
<210> 44
   <211> 30
   <212> DNA
   <213> Artificial sequence
<220>
   <223> Description of Artificial sequence: primer hPLOD2bRTF
<220>
   <221> misc_feature
   <222> (1)..(30)
<400> 44
   ttaaaggaaa gacactccga tcagagatga 30
<210> 45
   <211> 33
   <212> DNA
   <213> Artificial sequence
<220>
   <223> Description of Artificial Sequence: primer hPLOD2aRTR4
<220>
   <221> misc_feature
   <222> (1)..(33)
<400> 45
   tagccttcca aattcatgtc tattagaaat gta 33
<210> 46
   <211> 16
   <212> DNA
   <213> Artificial sequence
<220>
   <223> Description of Artificial sequence: antisense oligonucleotide (AON) 1
<220>
   <221> misc_feature
   <222> (1)..(16)
<400> 46
   cccatattcg gccctc 16
<210> 47
   <211> 15
   <212> DNA
   <213> Artificial Sequence
<220>
   <223> Description of Artificial sequence: antisense oligonucleotide (AON) 2
<220>
   <221> misc_feature
   <222> (1)..(15)
<400> 47
   ttccctttgt aaagt 15
<210> 48
   <211> 30
   <212> DNA
   <213> Artificial Sequence
<220>
   <223> Description of Artificial Sequence: PLOD2 forward primer
<220>
   <221> misc_feature
   <222> (1)..(30)
<400> 48
   ttaaaggaaa gacactccga tcagagatga 30
<210> 49
   <211> 28
   <212> DNA
   <213> Artificial Sequence
<220>
   <223> Description of Artificial Sequence: PLOD2 reverse primer
<220>
   <221> misc_feature
   <222> (1)..(28)
<400> 49
   aatgtttccg gagtagggga gtcttttt 28
<210> 50
   <211> 31
   <212> DNA
   <213> Artificial sequence
<220>
   <223> Description of Artificial Sequence: Beta 2-microglobulin forward primer
<220>
   <221> misc_feature
   <222> (1)..(31)
<400> 50
   tcttgtacta cactgaattc acccccactg a 31
<210> 51
   <211> 26
   <212> DNA
   <213> Artificial Sequence
<220>
   <223> Description of Artificial sequence: Beta 2-microglobulin reverse primer
<220>
   <221> misc_feature
   <222> (1)..(26)
<400> 51
   atccaaatgc ggcatcttca aacctc 26
<210> 52
   <211> 42
   <212> DNA
   <213> Artificial Sequence
<220>
   <223> Description of Artificial sequence: molecular beacon PLOD2
<220>
   <221> misc_feature
   <222> (1)..(42)
<400> 52
   cgtgcgcgtg ataaactgga tcctgatatg gctcttcgca cg 42
<210> 53
   <211> 36
   <212> DNA
   <213> Artificial Sequence
<220>
   <223> Description of Artificial Sequence: molecular beacon Beta 2-microglobulin
<220>
   <221> misc_feature
   <222> (1)..(36)
<400> 53
   cgtgccctgc cgtgtgaacc atgtgacttt ggcacg 36
<210> 54
   <211> 17
   <212> PRT
   <213> Artificial Sequence
<220>
   <223> Description of Artificial sequence: peptide containing a hydroxylatable lysine residue
<220>
   <221> SITE
   <222> (1)..(17)
<400> 54
   Gln Leu Ser Tyr Gly Tyr Asp Glu Lys Ser Thr Gly Gly Ile Ser Val
   1 5 10 15
   Pro
<210> 55
   <211> 12
   <212> DNA
   <213> Artificial Sequence
<220>
   <223> Description of Artificial sequence: wild type
<220>
   <221> misc_feature
   <222> (1)..(12)
<400> 55
   tctggtggtt at 12
<210> 56
   <211> 12
   <212> DNA
   <213> Artificial sequence
<220>
   <223> Description of Artificial Sequence: gly601/622→Val mutant
<220>
   <221> misc_feature
   <222> (1)..(12)
<400> 56
   tctgttggtt at 12
<210> 57
   <211> 12
   <212> DNA
   <213> Artificial Sequence
<220>
   <223> Description of Artificial Sequence: wild type
<220>
   <221> misc_feature
   <222> (1)..(12)
<400> 57
   tgtcccaact ga 12
<210> 58
   <211> 12
   <212> DNA
   <213> Artificial Sequence
<220>
   <223> Description of Artificial Sequence: family DR
<220>
   <221> misc_feature
   <222> (1)..(12)
   <223> /note="wherein N can be any nucleotide"
<400> 58
   tgtcccaant ga 12
<210> 59
   <211> 12
   <212> DNA
   <213> Artificial Sequence
<220>
   <223> Description of Artificial sequence: Thr608/629-> Ile mutant
<220>
   <221> misc_feature
   <222> (1)..(12)
<400> 59
   tgtcccaatt ga 12

## Description

### FIELD OF THE INVENTION

This invention is in the general field of modifying collagenous materials in order to increase or decrease their stability towards enzymatic degradation (in particular by proteinases). Furthermore, the invention is in the field of medical treatment of fibrotic conditions, and in the fields of diagnosis and/or monitoring of fibrotic processes. Furthermore, the invention is in the field of screening tests for substances that are potentially useful in the treatment of fibrotic conditions.

### BACKGROUND OF THE INVENTION

Fibrillar collagens (e.g. collagen type I, II, III, V and XI) consist of a triple helical domain, flanked by telopeptides at both the aminoterminal and carboxyterminal end of the molecule (N-telopeptide and C-telopeptide, respectively). Biosynthesis of collagen is a multistep process, resulting in modifications of both the triple helix and the telopeptides. One of the steps in the biosynthesis of collagen is hydroxylation of certain lysine residues in the triple helix and telopeptides by the enzyme lysyl hydroxylase (EC 1.14.11.4).

Extracellular collagen molecules aggregate spontaneously into microfibrils. Further stabilization of the molecules occurs by means of cross-links. Cross-linking is initiated by conversion of specific lysine (Lys) or hydroxylysine (Hyl) residues of the telopeptides into the aldehydes allysine and hydroxyallysine, respectively, by the enzyme lysyl oxidase (EC 1.4.3.13) [H.M. Kagan, 1994, Path. Res. Pract., 190: 910-919]. The aldehydes subsequently react with Lys or Hyl residues in the triple helix to give characteristic di-functional cross-links. These cross-links eventually mature into tri- or tetra-functional cross-links [D.R. Eyre, 1987, Meth. Enzymol., 144: 115-139; A.J. Bailey et al., 1998, Mech. Ageing Developm, 106: 1-56].

Two related routes for the formation of cross-links have been described, one based on allysine from the telopeptides, the other based on hydroxyallysine from the telopeptides. Each route results in chemically distinct cross-links. Examples of the hydroxyallysine cross-links are hydroxylysylpyridinoline (HP) and lysylpyridinoline (LP); the precursors of these cross-links are di-functional cross-links known in their reduced form as dihydroxylysinonorleucine (DHLNL) and hydroxylysinonorleucine (HLNL), respectively.

It is well known that the stability of collagen molecules in environments containing proteinases depends, amongst others, on the level of cross-linking. The stability of collagen molecules against proteinases can be enhanced by increasing the amount of cross-links. Cross-links can be enzymatically mediated cross-links and non-enzymatically mediated cross-links. The enzymatically mediated cross-links are generated by lysyl oxidase. Introduction of cross-links in a non-enzymatic way can be achieved by treating collagen with a variety of chemicals, such as aldehydes, epoxides, isocyanates, acyl azides, carbodiimides, reducing sugars (the so-called Maillard reaction), or by a variety of physical methods, such as irradition (e.g. short-wave UV irradiation) or dehydrothermal treatments. There is an extensive amount of literature and patents dealing with controlling the biodegradation time of collagen by means of enhancing collagen cross-linking by lysyl oxidase, chemicals or physical methods. Controlling the degradation time of collagenous materials is highly important, especially in the field of drug release and tissue engineering. The starting point to engineer a tissue is the design of a scaffold and a consideration of the kind of cells to be seeded into the scaffold. Scaffolds can also be used in various wound healing situations. Biodegradation of scaffolds is required to prevent longterm physical hindrance of the implant. The rate of degradation is dependent on the application and has to be in concert with tissue formation. Collagen is often used as a basis for the manufacturing of scaffolds. For a number of applications, non-crosslinked collagen cannot be used because of its susceptibility to decomposition by metalloproteinases before it can be remodelled into a resistant replacement. In such cases, a collagen scaffold is needed showing higher resistancies toward proteinases. Therefore, various methods have been developed to control the speed of degradation of collagen, such as the above mentioned chemical and physical methods.

A disadvantage of chemical and physical methods is that the position of the cross-links within the molecule cannot be controlled: cross-links are generated throughout the molecule. In addition, said cross-links can be intramolecular or intermolecular. Another disadvantage is that most chemical and physical methods partially denature the collagen molecules: denatured collagen is highly susceptible to proteolytic degradation. Other disadvantages are that certain cross-links show some toxicity, have immunogenic properties, adversily affect biomechanical properties, adversily affect cell/matrix interactions, or that the treatment enhances unwanted side-effects, such as calcification of the matrix.

These problems can be overcome by using lysyl oxidase: the formed aldehydes react with amino acids located at very specific positions within the triple helix. Furthermore, because the cross-links normally occur *in vivo*, the cross-links do not show toxicity or immunogenicity, and the treatment with lysyl oxidase does not result in a denaturation of collagen molecules.

In some cases, the durability of collagen molecules cross-linked by means of lysyl oxidase in proteolytic environments is not high enough, resulting in biodegradation times that are too short. In other cases, collagen cross-linked by lysyl oxidase show biodegradation times that are too long. The latter is for example the case in fibrosis. In fibrotic conditions an unwanted accumulation is seen of collagen molecules that is difficult to degrade by proteinases.

### SUMMARY OF THE INVENTION

A need exists for tools controlling the biodegradation time of collagenous materials based on the formation of naturally occurring cross-links. Here we show that collagen cross-linked by hydroxyallysine cross-links is more resistant toward proteolytic enzymes and more difficult to degrade than collagen cross-linked by allysine. Both cross-links occur *in vivo* and are generated by lysyl oxidase. Our approach is unique in that it controls the biodegradation time of collagens by controlling the lysyl hydroxylation level of the telopeptides. This is achieved by controlling the levels of telopeptide lysyl hydroxylase, the enzyme that hydroxylates the lysine residues located in the telopeptides.

The present invention also relates to the observation that the amount of hydroxyallysine cross-links is enhanced in fibrotic tissues, explaining in part the irreversibility of collagen accumulation in fibrosis.

Here we also show that the gene *PLOD2* encodes a telopeptide lysyl hydroxylase, making it possible to regulate the lysyl hydroxylation level of the telopeptides. Previously, it was not known whether the enzyme encoded by *PLOD2* hydroxylates the Lys in the telopeptides or the Lys in the triple helix of the collagen.

The invention has a broad applicability: it can be used for the preparation of collagenous materials used in tissue engineering or drug delivery but also in the field of medical treatments aimed at inhibiting fibrotic processes. In fibrotic tissues, a switch is seen in the telopeptide-lysyl-crosslink pathway toward the telopeptide-hydroxylysyl-crosslink pathway. Inhibition of the synthesis (transcription, translation) of the enzyme encoded by *PLOD2* and/or inhibition of the enzymatic activity of the PLOD2-encoded protein results in a collagen network that is predominantly cross-linked by means of allysine cross-links, a network that is more easy to degrade by proteinases. The subject invention also concerns new materials and methods for the detection of fibrotic processes based on the novel finding that the gene *PLOD2* encodes telopeptide lysyl hydroxylase and that this enzyme plays a key role in fibrosis.

The present invention provides methods for the preparation of collagenous materials exhibiting variable degradation times in environments containing proteolytic enzymes, based on modifying the hydroxylysine levels in the telopeptides.

The present invention provides a new method for selectively inhibiting the formation of hydroxyallysine cross-links, but not allysine-derived cross-links in wound healing and in other processes in which fibrosis occurs, thus increasing the proteolytic degradation rate of the assembled collagen network. The selectivity of the method is such that biosynthesis of allysine cross-links will not be compromised, thus resulting in collagen with favourable mechanical properties over non-cross-linked collagen. This specificity can be achieved by administration of an effective amount of a composition that selectively inhibits the activity or production of telopeptide lysyl hydroxylase but not lysyl oxidase.

The subject invention furthermore concerns methods for estimating or determining the amount of mRNA copies transcribed from the *PLOD2* gene as a means to monitor the onset and/or the progression of fibrosis/scarring. The invention further provides methods for measuring the amount of *PLOD2*-encoded enzyme in a sample by means of antibodies and/or aptamers or other means, and methods for determining telopeptide lysyl hydroxylase activity levels in a sample using one or more hydroxylatable sequences. Said methods are tools to diagnose or monitor fibrotic processes.

Additionally, the invention provides methods for measuring telopeptide lysyl hydroxylase activity in a test system designed to screen compounds exhibiting inhibitory properties towards the activity of the enzyme or antagonist properties with respect to the transcription of the *PLOD2* gene or the translation of the respective RNA template.

Further objects and advantages of the invention with respect to compositions capable of suppressing or repressing the synthesis (transcription and/or translation) of telopeptide lysyl hydroxylase and/or compounds capable of inhibiting the activity of said enzyme will be clear to one skilled in the art upon consideration of the following detailed description.

Examples demonstrate (1) that collagen cross-linked by allysine is easier to degrade by proteinases than collagen cross-linked by hydroxyallysine, (2) various mutations of the *PLOD2* gene in Bruck syndrome, showing that *PLOD2* is a telopeptide lysyl hydroxylase, (3) that increased *PLOD2* expression levels result in increased hydroxyallysine cross-link levels, (4) methods to measure mRNA levels of *PLOD2*, (5) that expression of *PLOD2* is highly increased in myofibroblasts, cells that mediate fibrosis, (6) formats of a high through-put assay designed to screen compounds exhibiting inhibitory properties towards telopeptide lysyl hydroxylase, (7) methods to prepare collagenous materials with varying degradation rates, (8) methods to increase telopeptide lysyl hydroxylase levels in cells in order to obtain collagen with high lysyl hydroxylation levels in the telopeptides, (9) methods to inhibit telopeptide lysyl hydroxylase in order to obtain collagen with low lysyl hydroxylation levels in the telopeptides.

### BRIEF DESCRIPTION OF THE DRAWINGS

**Fig. 1** shows the graphs depicting (**A**) the level of collagen degradation products (Hyp = hydroxyproline) as well as (**B**) the hydroxyproline to proline (Hyp:Pro) ratio in Bruck syndrome urine compared to urine from healthy subjects and urine from osteogenesis imperfecta (OI) patients. The data indicate that the degradation of bone collagen in Bruck syndrome, which is characterized by the almost complete absence of hydroxyallysine cross-links, is significantly elevated.
**Fig. 2** is a pedigree of a Bruck syndrome family (family DR and family PM) showing the genotypes in the region of homozygosity on chromosome 3 reference interval D03S1764-D03S1594 (164.6-168.3 cM) where the gene *PLOD2* is located. Black symbols denote affected individuals and striped symbols denote carriers of the disease. The haplotypes co-segregating with the disease are indicated with a black box. Both pedigrees provide evidence that *PLOD2* is the candidate gene for telopeptide lysyl hydroxylase.
**Fig. 3** is a graph showing the missense mutation found in Bruck syndrome family PM, resulting in a Gly→Val mutation in the sequence GGYENVPT (the mutated residue is underligned).
**Fig. 4** is a graph showing the missense mutation found in Bruck syndrome family DR, resulting in a Thr→Ile mutation in the sequence GGYENVPT (the mutated residue is underligned). Both Fig. 3 and 4 provide direct evidence that *PLOD2* encodes telopeptide lysyl hydroxylase.
**Fig. 5** is a graph showing the mRNA levels of *PLOD2B* (normalized against β2-microglobulin mRNA levels) in cultured human fibroblasts and cultured human myofibroblasts as determined by multiplex real-time PCR. The data show that *PLOD2B* is highly expressed in myofibroblasts, cells that play a key role in fibrotic processes.
**Fig. 6** is a graph showing the construct pDHPL2b.5 derived from the pMOSBlue vector used for the expression of recombinant *PLOD2B* in mammalian cells.
**Fig. 7** is a graph showing the expression of *PLOD2B* during osteogenic differentiation of human bone marrow cells. The amount of mRNA derived from *PLOD2B* is essentially the same in the various differentiation phases.
**Fig. 8** is a graph showing that the expression of *PLOD2B* is inhibited in fibroblasts and myofibroblasts by adding minoxidil to the culture medium.
**Fig. 9** is a graph showing that the expression of *PLOD2B* is increased in fibroblasts by adding TGF-β to the culture medium.

### DETAILED DESCRIPTION OF THE INVENTION

The invention provides a method for obtaining a collagenous matrix which comprises cross-linked collagen molecules wherein the resistance of said collagenous matrix against proteolytic degradation is controlled by controlling the ratio of hydroxyallysine cross-links to allysine cross-links in the collagenous matrix. The ratio of hydroxyallysine cross-links to allysine cross-links in the collagenous matrix may be controlled by controlling the lysyl hydroxylation level of the collagen telopeptides. The lysyl hydroxylation level of the collagen telopeptides may be controlled by controlling the level of telopeptide lysyl hydroxylase activity. The level of telopeptide lysyl hydroxylase activity may be controlled by controlling the expression of a PLOD2 gene or by controlling the telopeptide lysyl hydroxylase activity of a PLOD2 expression product. Preferably, the level of telopeptide lysyl hydroxylase activity is controlled without simultaneously affecting helical lysyl hydroxylase activity, although a simultaneous increase or decrease of helical lysyl hydroxylase activity may be accepted. More preferably, the level of telopeptide lysyl hydroxylase activity is controlled in such a way that lysyl oxidase activity is not affected. Thereby, the invention allows to achieve a modified ratio of hydroxyallysine cross-links to allysine cross-links in the collagenous matrix without significantly affecting the overall degree of cross-linking.

The invention comprises methods which are carried out *in vitro,* in particular methods for producing a collagenous matrix with a desirable resistance against proteolytic degradation or breakdown, i.e. a desirable biodegradation time, in particular a desirable high or low stability against proteolytic degradation. Such *in vitro* methods may involve the use of living cells for producing collagen, but may also be completely cell-free. The invention further comprises methods carried out *in vivo,* i.e. in living mammals, in particular in human beings, wherein a major application is in a treatment of fibrotic conditions.

Furthermore, the invention provides a method for diagnosing and/or monitoring the occurrence or state of a fibrotic process in a mammal comprising taking a sample (in particular a tissue sample) from said mammal, analyzing said sample to determine the expression level of a PLOD2 gene and comparing said expression level with a standard.

The invention also provides an assay (in particular a high-troughput assay) for screening compounds or compositions to determine their effect on telopeptide lysyl hydroxylase activity comprising contacting under enzymatically functional conditions a compound or composition to be tested with a PLOD2-encoded telopeptide lysyl hydroxylase enzyme and a suitable substrate for this enzyme, and determining the level of lysyl hydroxylation of the substrate compared to the level of lysyl hydroxylation of the substrate in the absence of the compound or composition to be tested. Such assay preferably further comprises separating, after said contacting of the compound or composition to be tested with a PLOD2-encoded telopeptide lysyl hydroxylase enzyme and suitable substrate for it, the substrate from the reaction mixture, reacting the substrate successively with an oxidizing agent, such as periodate, which is capable of oxidizing the hydroxyl group of Hyl to an aldehyde moiety, and a hydrazide dye, and measuring the fluorescence from the substrate.

Further, the invention provides an assay for screening compounds or compositions to determine their effect on telopeptide lysyl hydroxylase expression comprising growing cells expressing a PLOD2-encoded telopeptide lysyl hydroxylase enzyme in the presence of a compound or composition to be tested and determining the level of PLOD2 expression compared to the level of PLOD2 expression in the absence of the compound or composition to be tested.

### Definitions

Telopeptide lysyl hydroxylase refers to an enzyme that is capable of converting lysine residues of collagen telopeptides into hydroxylysine. As opposed to helical lysyl hydroxylase, telopeptide lysyl hydroxylase has a higher affinity for Lys located in the telopeptides than Lys located in the triple helical part of the collagen molecule.

In this invention, the term telopeptide lysyl hydroxylase refers to the biologically active enzyme encoded by the nucleic acid sequence of the *PLOD2* gene, or a nucleic acid sequence homologous to it. Said enzyme is derived from the complete mRNA sequence of *PLOD2,* a splice variant thereof, or a fragment of the *PLOD2* nucleic acid sequence (but still encoding a polypeptide displaying biological activity in the sense that it hydroxylates lysine residues in the telopeptides). The invention also encompasses *PLOD2* variants, i.e. differing in nucleotide sequence or even in amino acid sequence of the encoded polypeptide, but still encoding a polypeptide displaying activity in the hydroxylation of lysine residues in the telopeptides. Several *PLOD2* variants are known in the art. A preferred *PLOD2* variant is one having at least 90% amino acid sequence identity to the *PLOD2* amino acid sequence. As a result of the degeneracy of the genetic code, a multitude of polynucleotide sequences encoding telopeptide lysyl hydroxylase, some bearing minimal homology to the polynucleotide of *PLOD2,* may be produced. Thus, the invention contemplates each and every possible variation of polynucleotide sequences that could be made by selecting combinations based on possible codon choices.

Some examples of known *PLOD2* sequences can be found in M. Valtavaara et al. (1997, J. Biol. Chem., 272 (11): 6831-6834), H.N. Yeowell & L.C. Walker (1999, Matrix Biology, 18: 179-187), H. Ruotsalainen et al. (1999, Matrix Biology, 18: 325-329), and H. Ruotsalainen et al. (2001, Matrix Biology, 20: 137-146). Said publication by Yeowell & Walker described the presence of two splice variants of *PLOD2.* In this patent application, *PLOD2A* refers to the short form, whereas *PLOD2B* refers to the form of *PLOD2* containing exon 13A (located between exon 13 and exon 14).

The words "inhibits the activity or production of telopeptide lysyl hydroxylase" are used herein in a broad sense, in that they not only cover the actual inhibition of the enzyme as such, but also cover an inhibition of the transcription of the telopeptide lysyl hydroxylase gene, inhibition of the translation of mRNA derived from the telopeptide lysyl hydroxylase gene, and treatment with (a recombinant gene coding for) a mutated telopeptide lysyl hydroxylase or a fragment thereof that shows no activity towards telopeptides but that is competitive to endogenous telopeptide lysyl hydroxylase with respect to its natural substrate (collagen telopeptides).

The term "antagonist" as it is used herein, refers to a molecule which decreases the amount or duration of the effect of the biological activity of telopeptide lysyl hydroxylase. Antagonists may include proteins, nucleic acids, carbohydrates, antibodies, aptamers, or any other molecules which decrease the effect of telopeptide lysyl hydroxylase.

Fibrosis is a disorder or undesirable physical condition characterized by excessive deposition of collagen, resulting in scarring of the affected tissue(s).

### Collagen cross-linked by allysine shows a different molecular packing in fibrils and is more easily degraded compared to collagen cross-linked by hydroxyallysine

The bone collagen in Bruck syndrome lacks the hydroxyallysine cross-links of normal bone; the cross-links are replaced by allysine cross-links [R.A. Bank et al., 1999, Proc. Natl. Acad. Sci. USA, 96: 1054-1058]. To corroborate whether allysine cross-linked collagen molecules are more prone to degradation by proteolytic enzymes, both demineralized Bruck syndrome bone (containing allysine cross-links) and demineralized control bone (containing hydroxyallysine cross-links) were treated with pepsin dissolved in 0.5 M acetic acid. Demineralized Bruck syndrome bone treated with pepsin resulted in a release of 65% of the collagen molecules (compared to 5% of normal bone). Treatment of demineralized Bruck syndrome bone as well as demineralized control bone in 0.5 M acetic resulted in the solubilization of 7% and 1 % of the collagen molecules, respectively, indicating that the value of 65% released collagen is mainly due to the action of pepsin.

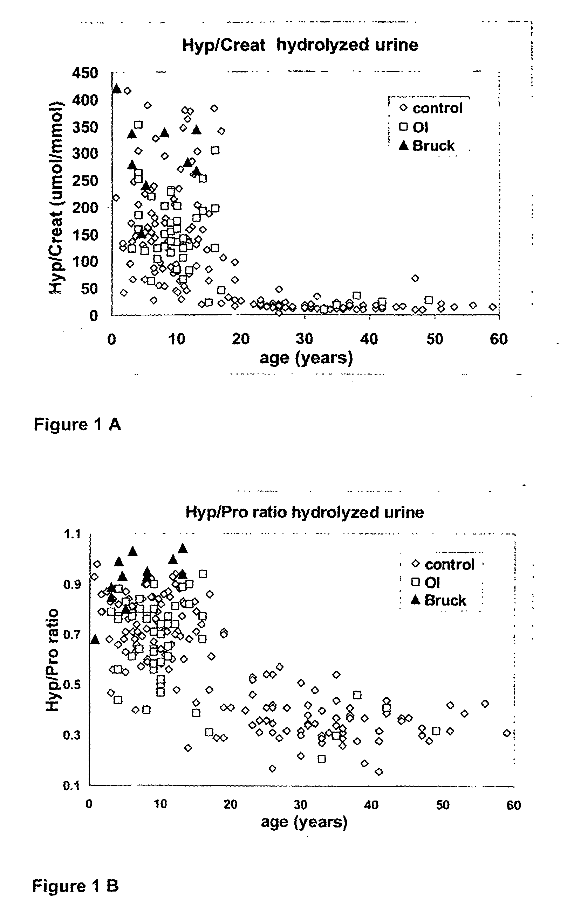

Additional evidence that allysine cross-linked collagen molecules are more prone to degradation by proteolytic enzymes was obtained by measuring hydroxyproline (Hyp) levels in urine of Bruck syndrome patients. We analyzed the urine of patients clinically diagnosed with Bruck syndrome (most patients have been described in the literature). The data presented in one of the examples show that the urinary level of hydroxyproline (µmol Hyp/mmol creatinine) in Bruck syndrome is higher than age-related controls or age-related osteogenesis imperfecta patients (Fig. 1A; *P*<0.001), showing that there is an increase of collagen degradation. The same can be concluded from the elevated hydroxyproline/proline ratio (Fig. 1 B; *P*<0.001). As most of the collagen degradation products in urine are derived from bone [M.S. Calvo et al., 1996, Endocrine Reviews, 14: 333-368], it is likely that Bruck syndrome patients show an increased turnover of bone collagen. Indeed, Bruck syndrome patients are osteoporotic, which is a further indication of increased bone collagen degradation. This phenomenon is attributable to the lack of hydroxyallysine cross-links in Bruck syndrome bone.

Interestingly, pepsinized collagen from Bruck syndrome bone (containing predominantly allysine cross-links) shows on SDS-electrophoresis two β-bands (β1,1 and β1,2 in a 1:2 ratio) whereas pepsinized collagen from normal bone (containing predominantly hydroxyallysine cross-links) shows on SDS-electrophoresis three β-bands (β1,1, β1,2 and β2,2 in a 1:1:1 ratio). The β-bands are two α-chains connected to one another by means of a di-functional cross-link. Cross-linking of collagen involves a few specific amino acids only. For steric and chemical reasons only these amino acids are able to react with each other when collagen molecules are correctly aligned. Consequently, variations in the packing arrangement must have an impact on the amount and nature of cross-links, or *vice versa.* In either case, cross-link patterns can be used as a marker to probe the alignment of intrafibrillar collagen molecules. It occurs to us that the difference in β-band patterns after pepsinization is the result of a different packing of the collagen molecules. J. Brinckmann et al. [1996, J. Invest. Dermatol., 107: 589-592] also disclosed that collagen molecules cross-linked by allysine show a packing arrangement within fibrils that is different from the packing of collagen molecules containing hydroxyallysine cross-links. We postulate that this difference in packing is causally involved in the observed increased degradation rate of allysine cross-linked collagen.

One of the examples describes a method for the preparation of collagenous compositions showing various ratios of hydroxyallysine over allysine cross-link ratios, thus showing various degradation rates towards proteinases.

In conclusion, collagen molecules cross-linked by allysine are both *in vitro* and *in vivo* more prone to proteolytic degradation. This explains the high excretion of collagen degradation products in urine of Bruck syndrome patients and consequently the osteoporotic status of the patients. *Vice versa,* one can state that collagen cross-linked by hydroxyallysine is more resistant towards proteinases. This observation provides new tools for the preparation of collagenous materials exhibiting various degradation times in environments containing one or more proteinases: the residence time of collagenous materials can be increased by increasing the hydroxylysine level in the telopeptides. It is remarkable, that the effects of the *type* of enzymatically mediated cross-links on the susceptibility of collagen to proteolytic enzymes has never been investigated and never been taken into consideration as a tool for increasing or decreasing the biodegradation time of collagenous matrices, despite the fact that it is known for a long time that the presence of hydroxyallysine-derived cross-links is indicative for irreversible collagen deposition (such as is seen in fibrosis, see below). A possible explanation for this ignorance is, that it was untill now not known how the conversion of lysine into hydroxylysine of telopeptides could be controlled. The conversion is mediated by the enzyme telopeptide lysyl hydroxylase. In order to control the conversion of lysine into hydroxylysine of the telopeptides, one needs to know the identity of said enzyme and its working mechanism. We will show in the following paragraph (6.2) that the gene *PLOD2* encodes for telopeptide lysyl hydroxylase.

### Sequencing of PLOD2 in Bruck syndrome patients revealed mutations, providing direct evidence that the gene PLOD2 encodes for telopeptide lysyl hydroxylase

Hydroxylation of Lys in the triple helix of collagen occurs exclusively on Lys present in the sequence Gly-X-Lys-Gly; a Lys in the X position is not hydroxylated. The hydroxylated Lys in the telopeptides is embedded in an entirely different amino acid sequence. In view thereof, the existence of two classes of enzymes has been postulated: a class of enzymes that converts the Lys in the triple helical sequence into hydroxylysine (Hyl) (helical lysyl hydroxylase) and a class of enzymes that is responsible for the conversion of Lys in the telopeptides into Hyl (telopeptide lysyl hydroxylase).

The most direct evidence that a class of telopeptide lysyl hydroxylases must exist has been derived from cross-link studies in Ehlers-Danlos type VI syndrome (EDS-VI) and Bruck syndrome. EDS-VI is a disease that is biochemically characterized by a hydroxylysine deficiency of the triple helix of collagen. EDS-VI patients show a normal level of pyridinolines in tissues (e.g. in collagen type I from bone) and a normal excretion level of pyridinolines in urine [B. Steinmann et al., 1995, Am. J. Hum. Genet., 57: 1505-1508; Açil Y, et al., 1995, J. Am. Acad. Dermatol., 33: 522-524]. Pyridinolines are cross-links derived from the hydroxyallysine route. Thus, in EDS-VI, despite the deficiency of Hyl in the triple helix, a normal amount of Hyl is present in the telopeptides. From this it follows, that the mutated gene in EDS-VI is coding for a helical lysyl hydroxylase. In Bruck syndrome, the opposite is seen: in bone, normal Hyl levels of the triple helix of collagen is seen, whereas hydroxyallysine-derived cross-links are virtually absent [R.A. Bank et al., 1999, Proc. Natl. Acad. Sci. USA, 96:1054-1058]. Thus, in Bruck syndrome, despite normal levels of triple helical Hyl, a deficiency of Hyl in the telopeptides is observed. By definition, the mutated gene in Bruck syndrome is a telopeptide Iysyl hydroxylase or a cofactor that is involved in TLH activity or a transcription factor that is involved in the expression/synthesis of the enzyme.

So far, three lysyl hydroxylase genes have been identified: *PLOD1*, *PLOD2* and *PLOD3*. The abbreviation PLOD is derived from procollagen-lysine, 2-oxoglutarate 5-dioxygenase (which is the systematic name of lysyl hydroxylase), whereas the 1, 2 and 3 indicates the sequence of discovery. Expression of the three PLOD genes shows a tissue-specific distribution [M. Valtavaara et al., 1997, J. Biol. Chem., 272: 6831-6834; M. Valtavaara et al., 1998, J. Biol. Chem., 273: 12881-12886; K. Passoja et al., 1998, Proc. Natl. Acad. Sci. USA, 95: 10482-10486; H. Ruotsalainen et al., 1999, Matrix Biol., 18: 325-329]. *PLOD2* also shows a tissue-specific splice variant [H.N. Yeowell & L.C. Walker, 1999, Matrix Biol., 18: 179-187]. Furthermore, there is some evidence at the DNA level that tissue-specific forms of *PLOD1* exist [H.N. Yeowell et al., 1994, J. Invest. Dermatol., 102: 382-384]. *PLOD1-3* have been expressed in a *Baculovirus* expression system; the proteins encoded by the cDNA exhibit activity towards the synthetic peptide containing the helical sequence IKGIKGIKG or ARGIKGIRGFSG. Although the specificity of the expressed gene products of *PLOD1-3* towards the different collagen types has so far not been investigated, the relatively low amino acid sequence homology between the different lysyl hydroxylases (around 50-60%) suggests differences in the substrate properties or functionality of said enzymes.

*PLOD1* is the gene that is mutated in Ehlers-Danlos type VI syndrome (EDS-VI) [J. Brinckmann et al., 1998, Arch. Dermatol. Res., 290: 181-186; H.N. Yeowell & L.C. Walker, 2000, Molec. Genet. Metab., 71: 212-224], a disease that is biochemically characterized by a hydroxylysine deficiency of the triple helix of collagen. In EDS-VI, a normal hydroxylation of the telopeptides is seen. Thus, the *PLOD1* gene encodes most likely for a triple helical lysyl hydroxylase.

So far, no disease has been associated with *PLOD2* or *PLOD3.* In a publication by R.A. Bank et al. [1999, Proc. Natl. Acad. Sci. USA, 96: 1054-1058], a Bruck syndrome family of Kurdish origin is shown where the defect (namely the absence of Hyl in the telopeptides of bone collagen) links to chromosome 17p12. This excludes *PLOD2* and *PLOD3* as candidate genes, as they are located on chromosome 3 and 7, respectively. The substrate specificity of the lysyl hydroxylase encoded by *PLOD2* and *PLOD3* is not known; the only substrate studies performed on both enzymes are incubations with a peptide displaying the helical sequence IKGIKGIKG or ARGIKGIRGFSG. Both enzymes were capable of hydroxylating these helical sequences [M. Valtavaara et al., 1997, J. Biol. Chem., 272: 6831-6834; M. Valtavaara et al., 1998, J. Biol. Chem., 273: 12881-12886; K. Passoja et al., 1998, Proc. Natl. Acad. Sci. USA, 95: 10482-10486]. From these data one would conclude that *PLOD2* and *PLOD3* encode for helical lysyl hydroxylases. In addition, *PLOD2* is expressed in skin [H.N. Yeowell & L.C. Walker, 1999, Matrix Biology, 18: 179-187; C. Wang et al., 2000, DNA and Cell Biology, 19: 71-77], a tissue where hydroxyallysine cross-links are present in extremely low levels. This would again suggest, that *PLOD2* encodes for a helical lysyl hydroxylase.

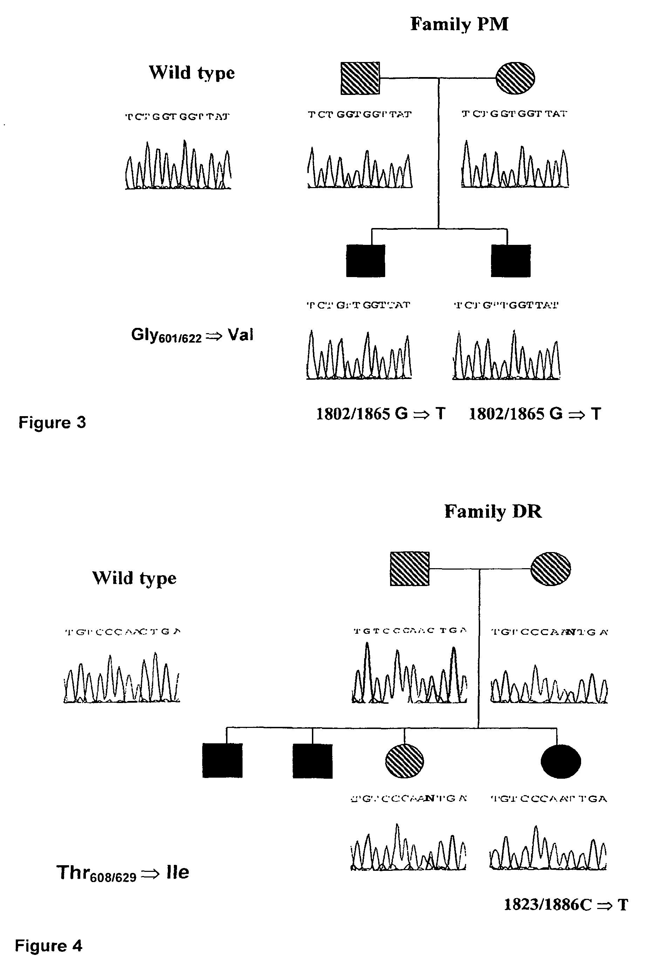

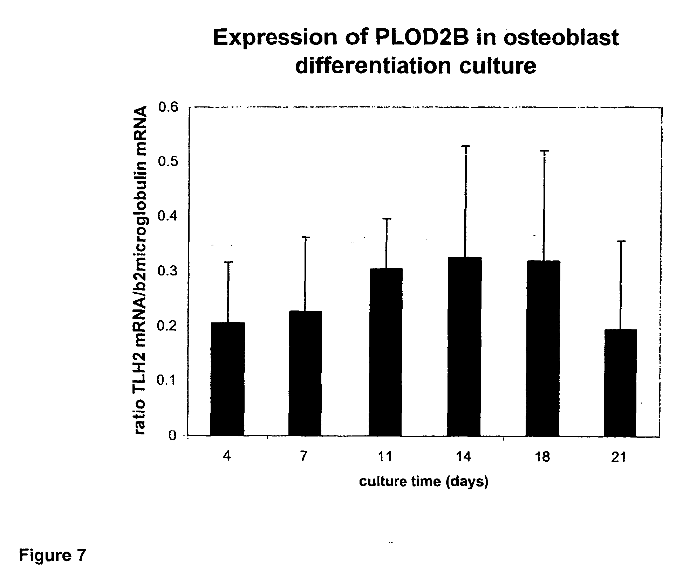

A study on (precursors) of osteoblasts provided data that the elevation of Lys hydroxylation in the telopeptides of type I collagen at day 16 (mineralization stage) coincide with a higher expression of *PLOD2* mRNA [K. Uzawa et al., 1999, J. Bone Miner. Res., 14: 1272-1280]. Based on these data, the authors hypothesized that the *PLOD2* gene might be involved in telopeptide lysyl hydroxylation. However, the data are not convincing: for the formation of pyridinoline cross-links a few days are required, and in this period PLOD2 expression is low, as is shown by said authors (their Fig. 3, second row, mRNA levels at the early differentiation stage = day 8). Their conclusion has therefore subsequently been ignored by other workers in the field. In fact, we have measured *PLOD2* levels in (precursors) of osteoblasts as well, and did not find an increase of *PLOD2* levels during osteoblastic differentiation, including the mineralization stage (Fig. 7). This was done with real-time PCR technology, which is much more reliable than the Northern blot technology used by Uzawa *et al*.

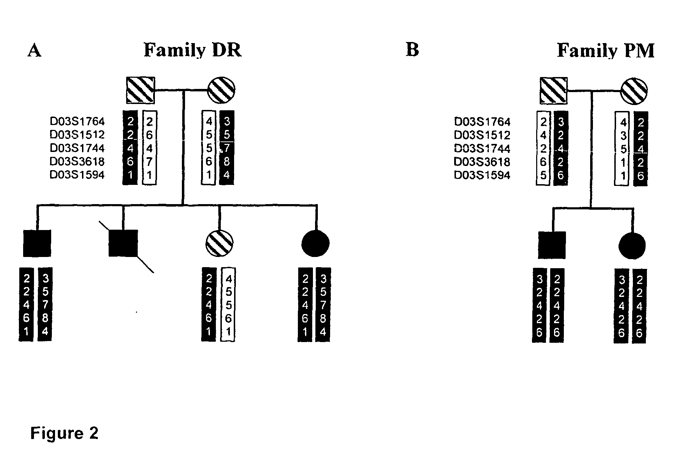

We have now investigated another consanguineous Bruck syndrome family (family PM) of Kurdish origin with 2 affected children but without healthy children (one of the children is case 8 of E.J. Breslau-Siderius et al., 1998, J. Pediatr. Orthop. B, 7: 35-38). In this pedigree an absence of linkage was found with our published markers of chromosome 17: haplotype analysis revealed that the affected children inherited different maternal chromosomes. The lysyl hydroxylase encoded by *PLOD2* is, according to Gene Map 98, located on chromosome 3 between the interval D3S1550 and D3S1306 (sex average 159.8 cM and 164.25 cM, respectively). Haplotype analysis was carried out of the parents and the two affected with the DNA markers D03S1764 (sex average 152.62 cM), D03S1512 (sex average 158.38 cM), D03S1744 (161.04 cM), D03S3618 (163.18 cM and D03S1594 (168.94 cM). The affected individuals were homozygous for D03S1512, D03S1744, D03S3618 and D03S1594 and haplotype analysis showed that the affected children inherited two identical copies of that chromosomal region (i.e. homozygous by descent) (Fig. 2B). Thus, a good linkage was found in this Bruck syndrome family and the chromosomal region where *PLOD2* is located. In conclusion, this Bruck syndrome family surprisingly provides genetic evidence that the lysyl hydroxylase encoded by *PLOD2* is telopeptide lysyl hydroxylase. A second Bruck syndrome family (family DR) also shows linkage to chromosome 3 (Fig. 2A).

The primary structure of *PLOD2* published in literature is primarily based on a cDNA sequence, meaning that the intron/exon boundaries are not know. *PLOD1* and *PLOD3* have 19 exons; intron/exon boundaries of these two genes are identical. As *PLOD2* is highly homologous with *PLOD1* and *PLOD3,* it is also expected that the exon/intron boundaries coincide with that of *PLOD1* and *PLOD3*. This was confirmed by analyzing the sequence of *Homo sapiens* chromosome 3 clones RP11-758I14 (GenBank code AC053539) and RP11-274H2 (GenBank code AC018369). In addition, an extra exon (not seen in *PLOD1* and *PLOD3*), designated as exon 13A, is an integral part of *PLOD2.* To provide direct evidence that *PLOD2* encodes for a telopeptide lysyl hydroxylase, exon 1 to exon 19 as well as exon 13A were individually amplified by means of a polymerase chain reaction (PCR) with primers located in the introns flanked at the 5' and 3' side of each exon using TaKaRa La Taq polymerase. This was done with genomic DNA (purified by means of standard methods) from Bruck syndrome patients, from the unaffected parents, and from commercially available genomic DNA obtained from a healthy control population (Roche Diagnostics, Cat.No. 1691112). Amplified exons of the expected size (as determined on 1% agarose gels) were separated from the free dNTP's and the polymerase by means of the Qiagen PCR purification kit. The purified exons were stained with fluorescent labeled dideoxy nucleotides (ddNTP's) in a cycle sequence PCR using the ABI PRISM^{™} BigDye Termination Cycle Sequencing Ready Reaction Kit and analyzed by means of capillary electrophoresis using the ABI PRISM^{™} 310 apparatus. For details of the used primers and PCR conditions for the amplification of the individual exons see one of the Examples. Sequence analysis of two Bruck syndrome patients of family PM revealed a nucleotide missense mutation resulting in a Gly6→Val mutation in the sequence GGYENVPT. This mutation was found on both alleles. Both parents were carrier for this missense mutation (see Fig. 3). In one Bruck syndrome patient of family DR a nucleotide missense mutation resulting in a Thr→Ile mutation in the sequence GGYENVPT. The patient was homozygous for the mutation while both parents and a healthy sister were heterozygous for this mutation (see Fig. 4). The two different point mutations found in family PM and DR were situated in a sequence that shows 100% amino acid homology between the different *PLODs* and between different species. This suggests an important role of this region in the function of lysyl hydroxylases in general and that of *PLOD2* in particular.

In conclusion, we have shown in this paragraph, much to our surprise in view of the evidence that pointed in another direction, that *PLOD2* encodes for a telopeptidyl lysyl hydroxylase. This satisfies a need in the art by providing a new tool in the preparation of collagenous compositions showing various resistance times towards proteinases as it is now possible to regulate the hydroxylysine levels in the telopeptides.

### Methods for increasing or decreasing hydroxyallysine cross-links over allysine cross-links by means of modulating telopeptide lysyl hydroxylase expression

The invention provides a method of treating a fibrotic condition in a mammal by administering to said mammal an effective amount of a compound or composition which reduces the lysyl hydroxylation level of collagen telopeptides and thereby results in a collagenous matrix having a decreased ratio of hydroxyallysine cross-links to allysine cross-links. As to the exact method of administration, dose to be administered and treatment protocol, the invention is not particularly restricted. These factors depend much on factors such as the active substance or composition to be administered, the tissue to be treated, the patient to be treated, the extent of the fibrosis, etc. For example, topical or subcutanous administration would be administration routes of choice when fibrosis occurs in skin or other easily accessible tissues. Administration by inhalation would be a preferable route of administration with fibrosis in lung tissue. Systemic administration, e.g. by intravenous injection or infusion, may be necessary when the fibrosis occurs in internal tissues, such as liver. The active agent may be coupled to a means for targeting to a particular site or tissue. The skilled person may determine for each individual case the best treatment strategy, with respect to route of administration, active substance or composition to be administered and dose and treatment protocol.

An approach to inhibit the conversion of telopeptide lysine into hydroxylysine for the preparation of collagenous materials with decreased hydroxylysine levels is the inhibition of transcription of the responsible telopeptide lysyl hydroxylase gene. A compound capable of silencing the promotor region of telopeptide lysyl hydroxylase is a potentially attractive compound for modifying the amount of hydroxyallysine cross-links over allysine cross-links. Compounds worthwhile to investigate are for example minoxidil and minoxidil analogues, compounds known to inhibit the transcription of the *PLOD1* gene [S. Murad et al., 1994, Arch. Biochem. Biophys., 308: 42-47].

A further approach to inhibit the synthesis of telopeptide lysyl hydroxylase is the inhibition of mRNA translation by means of antisense RNA (the transcription product of the DNA antisense strand, i.e. the strand that does not encode a protein). Transfection of the cells can be done with naked antisense RNA, antisense RNA emulsified in or coupled to carriers or vectors containing (parts of) the *PLOD2* DNA antisense strand.

Another approach to inhibit the conversion of telopeptidyl Lys into Hyl is inhibition of the activity of the enzyme telopeptide lysyl hydroxylase itself. Prolyl hydroxylase and lysyl hydroxylase have very similar catalytic properties (e.g. they share the same co-substrates). In addition, the inhibition patterns of prolyl and lysyl hydroxylases are very similar as well, but differences exists in some details, suggesting that significant differences exist between the catalytic sites of said hydroxylases. For example, *Kᵢ* values of the aliphatic and aromatic 2-oxoglutarate analogues (see below) are distinctively higher for lysyl hydroxylase encoded by *PLOD1* than for prolyl 3-hydroxylase and prolyl 4-hydroxylase. The data that have previously been obtained with respect to the inhibition of prolyl 3-hydroxylase, prolyl 4-hydroxylase and helical lysyl hydroxylase encoded by *PLOD1* can be used for a knowledge-based design of inhibitors of telopeptide lysyl hydroxylase. The assumption is strengthened by the observation, that the hydroxylation reaction carried out by the enzyme encoded by *PLOD2* can be followed, like that of *PLOD1*, by measuring the decarboxylation of 2-oxo-[1-¹⁴C]glutarate [Valtavaara et al., 1997, J. Biol. Chem., 272: 6831-6834].

The comparable reaction mechanism of of the enzyme encoded by *PLOD2* with that of the enzyme encoded by *PLOD1* provides a window with respect to the design of telopeptide lysyl hydroxylase inhibitors for those skilled in the art. What follows is a description of the reaction mechanism of the protein encoded by *PLOD2* based on previous findings for the protein encoded by *PLOD1*, followed by descriptions how this reaction mechanism can be inhibited. This should provide the skilled artisan sufficient guidance as the techniques closely parrallel experiments described in the past with respect to inhibition of the enzyme encoded by *PLOD1*. Lysyl hydroxylase acts on lysine in a reaction that requires ferrous ion (Fe²⁺), 2-oxoglutarate, molecular oxygen (O₂) and ascorbate. The 2-oxoglutarate is stoichiometrically decarboxylated during hydroxylation with one atom of the O₂ being incorporated into succinate while the other is complexed to the enzyme-bound ferrous ion. The latter results in a highly reactive iron-oxygen complex, ferryl ion. The oxygen atom of the ferryl ion is subsequently incorporated into the hydroxy group formed on the lysine residue, thereby converting the ferryl ion to the enzyme-bound ferrous ion. In the absence of a hydroxylatable substrate, lysyl hydroxylase is able to catalyze the decarboxylation reaction of 2-oxoglutarate in the presence of all the co-substrates [G. Tschank et al., 1994, Biochem. J., 300: 75-79]. In this so-called uncoupled reaction, the ferryl ion is converted to Fe³⁺ and OH⁻, and the Fe³⁺ ion remains bound to the active site, making the enzyme unavailable for new catalytic cycles until the Fe³⁺ is reduced into Fe²⁺ by ascorbate. The main role of ascorbate in the lysyl hydroxylase reaction *in vivo* is that of reactivating the enzyme after an uncoupled reaction [R. Myllylä et al., 1984, J. Biol. Chem., 259: 5403-5405]. As such, ascorbate plays a housekeeping role of restoring the iron constituent of the enzyme to the reduced state should it become oxidized adventitiously. The uncoupled reaction (and thus the oxidation of the iron) can be enhanced by peptides containing an unhydroxylatable sequence [D.F. Counts et al., 1978, Proc. Natl. Acad. Sci. USA, 75: 2145-2149; N.V. Rao & E. Adams, 1978, J. Biol. Chem., 253: 6327-6330].

The enzymatic reaction that converts telopeptide lysine into hydroxylysine can be used for the design of compounds that inhibit the activity of telopeptide lysyl hydroxylase without inhibiting lysyl oxidase. Examples of such potential inhibitors are:
- Compounds (such as aliphatic and aromatic structural analogues of 2-oxoglutarate) that bind to the subsite(s) of active site of the enzyme destined for the binding of 2-oxoglutarate. The inhibition of telopeptide Iysyl hydroxylase activity by said compounds is competitive with respect to 2-oxoglutarate.
- Compounds that chelate Fe²⁺ bound in the active site of telopeptide lysyl hydroxylase. The inhibition of telopeptide lysyl hydroxylase activity by said compounds is competitive with respect to the oxygen atom acceptor function of Fe²⁺ and/or with respect to the binding of Fe²⁺ to 2-oxoglutarate.
- Syncatalytic inactivation of telopeptide lysyl hydroxylase by anthracyclines or coumalic acid analogues.
- Syncatalytic inactivation of telopeptide lysyl hydroxylases by peptides containing an unphysiologic lysine derivate in a hydroxylatable position.
- Hydroxylatable peptides or peptidomimetics that are competitive with respect to the natural substrate (telopeptides) of telopeptide lysyl hydroxylase. A selective peptide or peptido mimetic is much less competitive with respect to the natural substrate of lysyl oxidase.
- Non-hydroxylatable peptides or peptido mimetics that are competitive with respect to the natural substrate (telopeptides) of telopeptide lysyl hydroxylase. A selective peptide or peptido mimetic is much less competitive with respect to the natural substrate of lysyl oxidase.
- Compounds that compete with collagen for the peptide substrate binding site on telopeptide lysyl hydroxylase. Possible examples are the organophosphate-like compounds like malathion and its oxidized derivative malaoxon, shown to inhibit helical lysyl hydroxylase [A. Samimi & J.A. Last, 2001, Toxicol. Appl. Pharmacol., 176: 181-186].

The uncoupled reaction of lysyl hydroxylase can be used for the design of compounds that inhibit the activity of telopeptide lysyl hydroxylase. Potent compounds are:
- Non-reducing ascorbate analogues that bind to the enzyme's active site but are not able to act as a specific alternative acceptor of ferryl oxygen. The presence of such a compound in the active site instead of an ascorbate (or an ascorbate analogue capable of reducing the ferryl ion) results in the inactivation of the enzyme by self-oxidation. The inhibition of telopeptide lysyl hydroxylase activity by said non-reducing ascorbate analogues is competitive with respect to ascorbate.
- Peptides or peptido mimetics with an unhydroxylatable sequence, capable of enhancing the uncoupled reaction of telopeptide lysyl hydroxylase. Said inhibitors result in increased levels of self-oxidized (non-active) levels of telopeptide lysyl hydroxylase.

A large number of studies have been published with respect to the inhibition of prolyl hydroxylase and helical lysyl hydroxylase by means of peptides or other compounds. What follows are a few selected examples of studies that can be used as a starting point in the design of inhibitors for telopeptide lysyl hydroxylase without affecting lysyl oxidase. Examples of syncatalytic inactivation by peptides: V. Günzler et al., 1988, J. Biol. Chem., 263: 19498-19504; K. Karvonen et al., 1990, J. Biol. Chem., 265: 8145-8419. Examples of syncatalytic inactivation by coumalic acid and anthracyclines: V. Günzler et al., 1987, Biochem. J., 242: 163-169; V. Günzler et al., 1988, Biochem. J., 251: 365-372. Examples of inhibitory competitive analogues of 2-oxoglutarate and ascobate: K. Majamaa et al., 1984, Eur. J. Biochem., 138: 239-245; K. Majamaa et al., 1985, Biochem. J., 229: 127-133; K. Majamaa et al., 1986, J. Biol. Chem., 261: 7819-7823. Examples of conformational requirements of lysyl hydroxylatable peptides: P. Jiang & V.S. Ananthanarayanan, 1991, J. Biol. Chem., 266: 22960-22967.

Apart from the knowledge-based design of inhibitors of telopeptide lysyl hydroxylase, a search can be performed with a library of compounds in order to find a compound that inhibitis the activity of telopeptide lysyl hydroxylase, but not lysyl oxidase.

A further example of inhibition of the catalytic properties of telopeptide lysyl hydroxylase is the use of antibodies. Antibodies directed against the enzyme and capable of blocking the active site or inhibiting the hydroxylation by telopeptide lysyl hydroxylase otherwise are potent inhibitors of the enzymatic reaction. These antibodies, preferably monoclonal, can be generated by immunization of mice with synthetic peptides or peptidomimetics containing stretches of amino acids of telopeptide lysyl hydroxylase, in particular sequences around the residues responsible for the binding of Fe²⁺ or 2-oxoglutarate to the enzyme. Promising candidate peptides or peptidomimetics for the generation of inhibiting antibodies are likely (but not necessarily) to be derived from the last 60 residues at the carboxy-terminal end of the enzyme (i.e. the region containing the conserved residues known to play a role in the catalytic properties of lysyl hydroxylase encoded by *PLOD1*). Monoclonal antibodies can also be generated by screening phage display libraries in their ability to block the activity of telopeptide lysyl hydroxylase. Antibodies not directed to the active site but directed against parts of the enzyme that are important for substrate binding are also potent inhibitors of the enzymatic reaction. In addition, antibodies directed towards the collagen telopeptides are also expected to inhibit the hydroxylation reaction of telopeptide lysyl hydroxylase by means of steric hindrance. Besides monoclonal or polyclonal antibodies, intracellular antibodies (intrabodies) can be used. Such intrabodies are encoded by engineered genes that are expressed within the cells of interest [I.J. Rondon & W.A. Marasco, 1997, Annu. Rev. Microbiol., 51: 257-283].

Alternatively, aptamers can be used to inhibit the reaction catalyzed by telopeptide lysyl hydroxylase. Aptamers are selected nucleic-acid binding species that bind to a target molecule with high affinity and specificity (the Latin word "aptus" means "to fit"). Nucleic acid aptamers that bind to telopeptide lysyl hydroxylase can be readily selected by the SELEX process, a technique for screening very large combinatorial libraries of oligonucleotides by an iterative process of *in vitro* selection and amplification [S.D. Jayasena, 1999, Clin. Chem., 45: 1628-1650]. The library typically contains 10¹⁴-10¹⁵ random DNA sequences flanked by two fixed sequence regions. Sequences that bind to the target molecule (the latter being fixed on a solid support) are separated from sequences that do not bind by a simple washing step. The population of sequences bound to the target is amplified by PCR using primers to the two fixed sequence regions. The enriched library can be used for the next selection/amplification cycle. The enrichment efficieny of high-affinity binders is governed by the stringency of selection at each round. The enriched library is cloned and sequenced to obtain the sequence information of each member. The generated aptamers have to be screened for their ability to inhibit the target enzyme by means of an assay designed to measure the activity of telopeptide lysyl hydroxylase.

In yet another approach to inhibit the conversion of telopeptide lysine into hydroxylysine is the delivery of constructs containing a telopeptide lysyl hydroxylase that is able to bind telopeptides but is not capable to convert the lysine of the telopeptides into hydroxylysine. Such an exogenous telopeptide lysyl hydroxylase is competitive to endogenous telopeptide lysyl hydroxylase with respect to its natural substrate (telopeptides). For said construct telopeptide lysyl hydroxylase can be used that is mutated by means of site-directed mutagenesis. Candidate residues for site-directed mutagenesis are the mutated residues found in Bruck syndrome. Other candidate residues for site-directed mutagenesis are the residues that are needed for the full activity of helical lysyl hydroxylase [A. Pirskanen et al., 1996, J. Biol. Chem., 271: 9398-9402] and that are conserved in the lysyl hydroxylase encoded by *PLOD2.* Especially interesting are the three ligands needed for the binding of Fe²⁺ to the catalytic site of lysyl hydroxylase (for helical lysyl hydroxylase encoded by *PLOD1* this is His-638, Asp-640 and His-690; numbering begins with the first residue in the processed polypeptide) or the residue that is responsible for the binding of 2-oxoglutarate to the enzyme (which is Arg-700 for the helical lysyl hydroxylase encoded by *PLOD1*) [K. Passoja et al., 1998, FEBS Letters, 434: 145-148]. The same residues are conserved in *PLOD2.* Residues that are also of potential interest are glycosylated Asn-X-Thr/Ser sequences: glycosylated lysyl hydroxylase encoded by *PLOD1* has a higher activity than its de-glycosylated counterpart [R. Myllylä et al., 1988, Biochem. J., 253: 489-496]. It thus seems that asparagine-linked oligosaccharides are required to obtain maximum lysyl hydroxylase activity. Two potential attachment sites for asparagine-linked oligosaccharides of the lysyl hydroxylase encoded by *PLOD2* have an homologous location in the sequence of the helical lysyl hydroxylase encoded by *PLOD1*. Other residues of potential interest are the cysteines of lysyl hydroxylase 2. These examples should not be construed as limiting.

A further embodiment of the subject invention for inhibiting the conversion of telopeptide lysine into hydroxylysine is the administration of an effective quantity of peptides comprising a hydroxylatable Lys. Peptides which act as enzyme substrates reduce the levels of enzyme available for hydroxylating collagen telopeptides. A method for the delivery of such peptides into the cells is the delivery of plasmid constructs containing the nucleotide sequence encoding for such peptides.

### Pathological levels of hydroxallysine cross-links results in an unwanted accumulation of collagen molecules (fibrosis)

In abnormal wound healing of the skin, such as in hypertrophic scarring, large amounts of hydroxyallysine-derived cross-links (such as DHLNL) are seen [A.J. Bailey & N.D. Light, 1985, Ciba Found. Symp., 114: 80-96]. A predominance of DHLNL is also found in collagen produced after wounding of the corneal stroma; the resulting scar shows markedly increased levels of hydroxyallysine derived cross-links at the expense of allysine cross-links [D.J. Cannon & S. Cintron, 1975, Biochim. Biophys. Acta, 412: 18-25]. The pioneering studies on elevated hydroxyallysine-derived cross-links in abnormal scarring were later confirmed, followed by reports on increased hydroxyallysine-derived cross-links in other (mainly fibrotic) disorders, such as various lung diseases (respiratory distress syndrome, idiopathic pulmonary fibrosis, hypersensitivity pneumonitis, respiratory bronchiolitis, silicosis and bleomycin-induced lung fibrosis), chronic adriamycin nephropathy (an experimental model resulting in non-immunologic glomerulosclerosis and interstitial fibrosis), infarct scar of the myocardium, joint contractures, vessel luminal narrowing, lipodermatosclerosis, annulo-aortic ectasia, fibrotic lesions of Dupuytren's disease, skin of patients with lipoid proteinosis, diabetes, skin fibrosis due to chromoblastomycosis infection, skeletal muscle injury, tendon hypertrophy and various liver diseases such as in alveolar echinococcosis (a dense and irreversible fibrosis), hepatocellular carcinoma, alcoholic cirrhosis or cirrhotic livers induced by viral hepatitis or by *Schistosoma mansoni*. From this abundant amount of data on elevated hydroxyallysine cross-link levels in fibrotic tissues we can conclude that one of the characteristics of fibrotic lesions is an upregulation of telopeptide lysyl hydroxylase.

In recent years, it has been suggested that the relative (and absolute) amount of hydroxyallysine cross-links are adequate biomarkers for the accumulation of collagen in lung and liver fibrosis [J.A. Last et al., 1990, Am. Rev. Respir. Dis., 141: 307-313; S. Ricard-Blum et al., 1995, Parasite, 2: 113-118]. As the same is seen in other fibrotic tissues (such as skin and kidney), it was actually stated that "It is possible that organ fibrosis is a unique process ultimately associated with a change in cross-linking whereby the proportion of the allysine cross-links decreases in favor of the hydroxyallysine-derived cross-links" [J. Brinckmann et al., 1996, J. Invest. Dermatol., 107: 589-592]. Thus, hydroxyallysine cross-links are implicated in the pathogenesis of fibrosis. As a matter of fact, hydroxyallysine cross-link levels might be an important criterion in assessing the irreversibility of fibrosis. The validity of this statement is strengthened by cross-link patterns seen in acute (self-limiting) and progressive forms of fibrosis. Collagen produced in response to an injury of skin is initially stabilized by DHLNL, a cross-link derived from hydroxyallysine. In the early stages of wound healing, the collagen of both forms of fibrosis possess DHLNL as the major cross-link, but after a few months there is an approximately equal proportion of HLNL. Subsequently, acute and progressive fibrosis follow a different course. In hypertrophic scars, a progressive form of skin fibrosis, the 1:1 ratio of the two cross-links is retained. In contrast, the cross-link pattern in the self-limiting form of fibrosis gradually reverts to normal, i.e. there is a disappearance of hydroxyallysine derived cross-links and replacement by allysine derived cross-links. In addition, the HLNL of old hypertrophic scars is derived from hydroxyallysine, and therefore stabilized by undergoing the Amadori rearrangement. The HLNL of normal scars is like normal dermis in being almost entirely derived from allysine [A.J. Bailey & N.D. Light, 1985, Ciba Found. Symp., 114: 80-96].

The data mentioned in this paragraph provides additional evidence for our statements that collagen cross-linked by means of hydroxyallysine derived cross-links is more difficult to degrade than collagen cross-linked by means of allysine derived cross-links. The data indicate that the type of cross-links provides a mechanism for regulating the rate of collagen catabolism: collagen with hydroxyallysine cross-links is less susceptible to proteolytic degradation than collagen cross-linked by allysine residues. Clearly, the production of collagen containing telopeptide lysine instead of telopeptide hydroxylysine would be beneficial for treating fibrotic conditions. Inhibition of telopeptide lysyl hydroxylase (to enhance the formation of allysine cross-links at the expense of hydroxyallysine cross-links) is an attractive way for interfering with a fibrotic respons by reducing the amount of hydroxyallysine derived cross-links over allysine-derived cross-links, making the collagen more susceptible to proteolytic degradation. In the next paragraph we will show that *PLOD2,* encoding telopeptide lysyl hydroxylase, is indeed highly expressed in fibrotic cells.

### Fibrotic cells contain high levels of PLOD2 mRNA

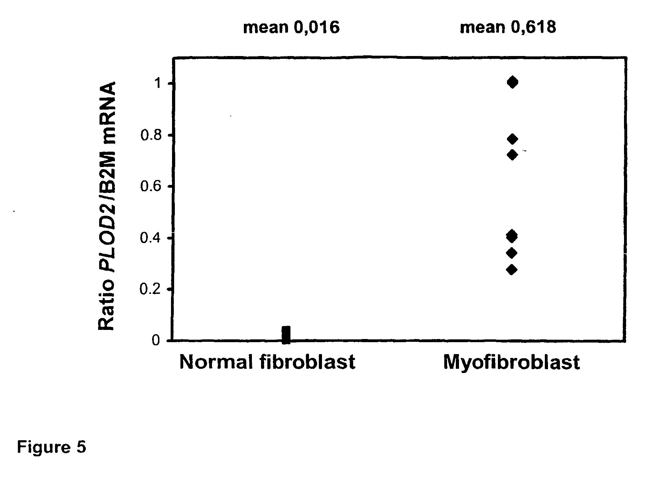

Clearly, fibroblasts in fibrotic tissues (the so-called myofibroblasts) are fundamentally different from that of normal fibroblasts: myofibroblasts synthesize collagen with increased hydroxylysine levels of the telopeptides. Such collagen is destined to become irreversibly incorporated into the collagen network of the tissue by means of hydroxyallysine cross-links. The fact that HP, the maturation product of DHLNL, might serve as a permanent marker of a fibrotic event indicates that such cross-linked collagen molecules show a low to negligible rate of degradation. We have concluded in the previous paragraphs that hydroxylation of the telopeptide lysine is controlled by telopeptide lysyl hydroxylase, and that *PLOD2* encodes for telopeptide lysyl hydroxylase. Therefore, *PLOD2* must be overexpressed in myofibroblasts. We have checked this by measuring mRNA levels of *PLOD2* in normal fibroblasts and in myofibroblasts by means of real-time PCR techniques. To strengthen our conclusion, that telopeptide lysyl hydroxylase is a key enzyme in fibrosis, we also measured mRNA levels of *PLOD1, PLOD3,* and collagen type I (*COL1A1*). Figure 5 shows that myofibroblasts have in mean a 40-fold increase of *PLOD2* mRNA levels compared to fibroblasts. In contrast, only a 4-fold increase was observed in *PLOD1* and *COL1A1* mRNA levels. *PLOD3* levels were in most cases not elevated. The data indicate that telopeptide lysyl hydroxylase is highly upregulated in fibrotic tissues.

### Measurement of PLOD2 mRNA, teloptide lysyl hydroxylase protein level or telopeptide lysyl hydroxylase activity level as a tool to diagnose or monitor fibrotic processes

The invention provides a method for diagnosing and/or monitoring the occurrence or state of a fibrotic process in a mammal comprising taking a sample from said mammal, analyzing said sample to determine the expression level of a PLOD2 gene and comparing said expression level with a standard. Suitable samples are tissue samples, in particular from tissues at risk for, or involved in, fibrotic processes. Suitable standards represent the expression level of a PLOD2 gene in normal tissues, i.e. not affected by fibrotic processes.

Detection of transcriptional acitivity of the gene *PLOD2* may be achieved by assaying the level of mRNA derived from *PLOD2* (e.g., by Northern blot analysis, mRNA analysis by competitive PCR or real time PCR), the protein level of telopeptide lysyl hydroxylase (e.g., by Western blot analysis, or by immunoassays such as ELISA), or the level of functional enzymatic activity of telopeptide lysyl hydroxylase (e.g., by means of hydroxylatable peptides).

In the first approach, transcriptional activity of the *PLOD2* coding region can be assessed by hybridization assays. For example, RNA can be isolated and analyzed by Northern blot using a probe homologous to the telopeptide lysyl hydroxylase coding sequence or particular portions thereof. Estimation of mRNA levels can also be achieved by using PCR-based technologies, such as the conversion of mRNA to cDNA by reverse transcriptase, followed by e.g. competitive PCR or real-time PCR. A description of a real-time PCR to measure *PLOD2* levels is given in one of the Examples.

In the second approach, the expression of the enzyme product (reflecting translational activity) can be assessed immunologically, for example by Western blots, immunoprecipitation, immunoassays such as enzyme-linked immunoassays and the like. The antibodies can be polyclonal, monoclonal, or chimeric of nature. The antibodies can be obtained by immunization of animals with the native or denatured enzyme or fragments thereof (such as synthetically manufactured peptides). Alternatively, the antibodies can be raised by means of the phage display method. The enzyme product can also be assessed by means of aptamers based on DNA/RNA sequences [S.D. Jayasena, 1999, Clin. Chem., 45: 1628-1650].

In the third approach, the expression of the enzyme product can be assessed by methods reflecting the enzymatic activity of the enzyme. Such methods are described in the next chapter.

### High through-put assays for measuring telopeptide lysyl hydroxylase activity

The invention provides an assay for screening compounds or compositions to determine their effect on telopeptide lysyl hydroxylase activity comprising contacting under enzymatically functional conditions a compound or composition to be tested with a PLOD2-encoded telopeptide lysyl hydroxylase enzyme and a suitable substrate for this enzyme, and determining the level of lysyl hydroxylation of the substrate compared to the level of lysyl hydroxylation of the substrate in the absence of the compound or composition to be tested.

The following description illustrates a method which can potentially lead to a high through-put assay for measuring the activity of telopeptidyl lysyl hydroxylase. Such an assay can be used for the screening of compounds in order to determine their antagonist properties, or as a tool to diagnose or monitor fibrotic processes.

A biotinylated peptide containing a telopeptide-like sequence containing at least one hydroxylatable Lys is subjected to incubation with telopeptide lysyl hydroxylase. The resulting mixture is treated with periodate or other suitable oxidizing agent. The 2-amino alcohol of Hyl in peptides has been shown to undergo rapid periodate oxidation to create an aldehyde [D.D. van Slyke et al., 1941, J. Biol. Chem., 141: 681-705]; the same occurs with peptides with a N-terminal Ser or N-terminal Thr. By designing a peptide substrate lacking N-terminal Ser or Thr in the peptide, only peptides containing a Hyl will form an aldehyde by means of periodate treatment. The aldehyde is then reacted with a tagging group, for instance in the form of a hydrazide, R'CONHNH₂ to form a hydrazone, R'CONHN=CH-peptide. R' can be any variety of useful groups, such as Lucifer Yellow, Texas Red or Cascade Blue. The biotinylated peptide can be separated from the remaining reaction mixture by immobilization based on the strong interaction of biotin with avidin or streptavidin. Other options are e.g. the use of peptides containing a repetitive His-sequence in combination with Ni²⁺ carriers or SAMA-peptides in combination with maleimide carriers. Measuring the fluorescence of the tag attached to Hyl provides information about the conversion of the Lys of the peptide into Hyl by telopeptide lysyl hydroxylase. Tags can also be used that are suitable for colorimetric or radioactive measurements.

It should be stressed, that the oxidation of Hyl by periodate results in the stoichiometric release of formaldehyde. 4-Amino-3-pentene-2-one (fluoral P) is reported to selectively react with formaldehyde to form a fluorescent dihydropyridine product [H. Tsuchiya et al., 1994, Analyst, 119: 1413-1416], even when aldehyde concentrations 2000 times that of detectable formaldehyde are present. Thus, measuring the release of formaldehyde is another method to provide information whether the Lys of the peptide is converted into Hyl by telopeptide lysyl hydroxylase.

In yet another format, energy-transfer substrates can be prepared for measuring whether a Lys or a Hyl is present in the peptide. In this type of substrate, two chromophoric groups that form a Forster energy donor-acceptor pair are placed at opposite ends of a peptide. The fluorescence emission of the donor overlaps the absorption spectrum of the acceptor, causing the fluorescence of the donor to be quenched while the substrate remains intact. When the intervening peptide region is cleaved by a proteinase, relief of the strongly distance-dependent quenching provides the means to follow this activity. *In concreto*, a energy-transfer substrate (a peptide containing a telopeptide-like sequence containing at least one hydroxylatable Lys and containing a fluorophore and a quencher) is subjected to telopeptide lysyl hydroxylase followed by digestion with trypsin or lysyl endopeptidase. The latter cleaves the peptide bond at the C-terminal end of Lys; trypsin cleaves the peptide bond on the C-terminal side of Lys and Arg. Hydroxylation of Lys residues reduces their susceptibility to digestion by trypsin or lysyl endopeptidase [M.S. Molony et al., 1998, Anal. Biochem., 258: 136-137]. In this format, diminished increase of fluorescence is seen at a given time when the Lys is converted into Hyl.

Other high through-put formats may be based on antibodies or aptamers recognizing the non-hydroxylated peptide but not the hydroxylated peptide or *vice versa.*

### EXAMPLES

### Example 1

### Pepsin digestion of normal and Bruck syndrome bone shows differences in collagen degradation by proteolytic enzymes and differences in collagen packing

Bruck syndrome and normal bone was demineralized at 4°C with 0.5 M EDTA, 0.05 M Tris-HCl, pH 7.5 over 2 weeks. Demineralized Bruck syndrome and normal bone was incubated for 24 h at 4°C with 0.5 M acetic acid (HAc) or with pepsin (enzyme:substrate ratio 1:10, w/w) in 0.5 M HAc. The solubilized collagen (present in the supernatant) was separated from the insoluble collagen matrix (containing the non-solubilized collagen) by means of centrifugation; both were hydrolyzed with 6 M HCl in Teflon sealed glass tubes (110°C, 20-24 h). The amount of the collagen-specific amino acid hydroxyproline (Hyp) was measured with reversed-phase high-performance liquid chromatography [R.A. Bank et al., 1997, Matrix Biol., 16: 233-243]. The amount of solubilized collagen was expressed as a percentage of total collagen using the equation Hypₛᵤₚ/(Hypᵣₑₛ + Hypₛᵤₚ) x 100 % where Hypₛᵤₚ is the amount of Hyp in the supernatant and Hypᵣₑₛ the amount of Hyp in the residual tissue.

Collagen solubility in 0.5 M acetic acid was 7% and 1% for Bruck syndrome and normal bone, respectively. Treatment with pepsin resulted in the release of 65% of the collagen from Bruck syndrome bone, whereas treatment with pepsin resulted in the release on only 5% of the collagen from normal bone. Clearly, the collagen molecules in the fibrils in Bruck syndrome bone are more prone to degradation by proteinases. As the triple helix of collagen type I in Bruck syndrome is normally modified, the increased release of collagen by pepsin of Bruck syndrome bone is due to the replacement of hydroxyallysine cross-links by allysine cross-links.

The pepsin-solubilized collagen of normal bone and Bruck syndrome bone was subjected to SDS-polyacrylamide gel electrophoresis and subsequently stained with Coomassie Brilliant Blue; the staining pattern of normal bone revealed three β-bands (β1,1, β1,2 and β2,2 in a 1:1:1 ratio) whereas the staining pattern of Bruck syndrome bone revealed two β-bands (β1,1 and β1,2 in a 1:2 ratio). The β-band patterns show that there are differences in the packing of intrafibrillar collagen molecules between normal and Bruck syndrome bone.

### Example 2

### Hydroxyproline measurements in urine revealed that in vivo degradation of bone collagen is enhanced in Bruck syndrome

Urine samples (500 µl) were hydrolyzed in an oven (110 °C; 20-24 h) with 500 µl 12 M HCl in 5 ml Teflon sealed glass tubes. After drying (SpeedVac, Savant), the hydrolysates were dissolved in 1 ml water, and diluted 400 times in 0.1 M borate buffer pH 9.5. A 200 µl sample was mixed with 25 µl o-phthaldialdehyde (OPA) reagent (30 mg OPA + 15 µl β-mercaptoethanol in 1 ml acetone) and reacted for 1 min at room temperature. The reaction mixture was mixed with 25 µl iodoacetamide (80 mg/ml acetone) and incubated at room temperature for at least 30 sec to remove excess β-mercaptoethanol. Subsequently, the secondary amino acids hydroxyproline (Hyp) and proline (Pro) were derivatized with 50 µl 6 mM 9-fluorenylmethyl chloroformate in acetone for 5 min at room temperature. Immediately thereafter, the sample was extracted twice with 700 µl diethyl ether to terminate the reaction and to remove excess reagent. After the addition of 400 µl 25% (v/v) acetonitrile in 0.1 M borate buffer pH 8.0, a 50 µl aliquot of the derivatization mixture was injected into the HPLC system. In this way, 0.0208 µl of the original urine sample was applied onto the column. Reversed-phase chromatography of the samples on a Micropak ODS-80TM column (150 mm x 4.6 mm; Varian, Sunnyvale, CA, USA) was performed as described elsewhere [R.A. Bank et al., 1996, Anal. Biochem., 240: 167-176]; Hyp calibration was performed with an amino acid standard for collagen hydrolysates obtained from Sigma (A-9531; St. Louis, MO, USA).

Fig. 1A-B shows the urinary excretion of Hyp and the Hyp/Pro ratio in Bruck syndrome patients *versus* controls as a function of age. All patients show a high excretion of Hyp, indicating a high degradation rate of collagen. As most of the collagen degradation products in urine are derived from bone [M.S. Calvo et al., 1996, Endocrine Reviews, 14: 333-368], the data show that Bruck syndrome patients have an increased turnover of bone collagen. This is due to the replacement of hydroxyallysine cross-links by allysine cross-links in Bruck syndrome bone, making the collagen network more susceptible towards proteinases.

### Example 3

### Mutation analysis of the PLOD2 gene of Bruck syndrome patients reveals that PLOD2 is telopeptide lysyl hydroxylase

Amplification of the individual exons of *PLOD2* was carried out with the primers presented in table 1. The PCR mix consisted of 5 µl TaKaRa 10x PCR buffer, 8 µl of of mixture containing the four dNTPs (2.5 mM each), 1 µl forward primer and 1 µl reverse primer (50 µM each), 0.5 µl TaKaRa La Taq polymerase (50 U/µl), 2.5 µl DNA (200 ng/µl), 5 µl DMSO and 27 µl H₂O. Thirtyfive cycles of amplification were carried out. Each cycle consisted of: 1 min denaturation at 94 °C, 0.5-1.5 min at 56-60 °C for annealing (see table for the exact condition per primer set) and 1 min at 68 °C for the extension. The amplified exons were purified with the Qiagen PCR purification kit; 10 ng of each exon was subjected to a cycle sequence PCR using the ABI PRISM^{™} BigDye Termination Cycle Sequencing Ready Reaction Kit and analyzed by means of capillary electrophoresis using the ABI PRISM^{™} 310 apparatus.

**Table 1:**

| **Primers used for the amplification of the individual exons of *PLOD2*** | | | | |
|---|---|---|---|---|
| **Exon** | **Forward primer** | **Reverse primer** | **Product length** | **Annealing temperature** |
| Promoter | **5' CTCCCAAAGCTAAGTGCAGG 3'** | 5' AGACAGGGATTCCAGGGGT 3' | 524 bp | 56°C 30" |
| 1 | **5' GTCTCTGCGTTCTCGCGAGA 3'** | 5' AAGGGCTGTTGGATGAATGAAC 3' | 260 bp | 56°C 1'30" |
| 2 | **5' TGAGGTCTCAATTACTGTAGTGA 3'** | 5' CTTCCTTGTGAGGATTACAGATT 3' | 272 bp | 56°C 1'30" |
| 3 | **5' GTACTGTTCAAGTTGATGATGTC 3'** | 5' GCCACCGTGCCCAACCATATT 3' | 334 bp | 56°C 30" |
| 4 | 5' ATGGTTTATGTGCCTAGATTCTGA 3' | **5' GGAACACCAACTCACATAATACA** 3' | 390 bp | 56°C 1'30" |
| 5 | 5' **TTCTTTCATGGTGAGCTGTGA** 3' | 5' TGATATCCAGCCAGGTGACA 3' | 442 bp | 56°C 30" |
| 6 | **5' GCAACTATCGCAGTTTCTACCT 3'** | 5' CCAAATGGACATAACAAAGGAAAG 3' | 331 bp | 56°C 30" |
| 7 | 5' **CACATACACACACAGACACACG** 3' | 5' AAAGGCTATCACTCTGCTGAGG 3' | 379 bp | 64°C 30" |
| 8 | **5' TAAAGGAATATACCTGCTGCAGA 3'** | 5' ATTCCACTTACATCTACTGCAGA 3' | 234 bp | 56°C 1'30" |
| 9 | 5' TTTCAAGTGTTAGAGAACTGCCA 3' | **5' CCACTGAACTTAACCCAATGAAT** 3' | 392 bp | 56°C 1'30" |
| 10 | 5' TCTAAGATTTCTAGGCTACAGGC 3' | **5' GTTGGCTACTGCATACGCAAAC** 3' | 633 bp | 60°C 30" |
| 11 | 5' **CAGAAAAGTATGCTAGAGAACCA** 3' | 5' GTAGAACATAACTAAGTTCCCTC 3' | 336 bp | 56°C 1'30" |
| 12 | 5' CAGGTTTGTTGAATGAGCTTTCT 3' | **5' AGGATTCCAAGTGGTCTTGGG** 3' | 398 bp | 60°C 1'30" |
| 13 | 5' GGGGCAGTGGTTTATCTCCTA 3' | **5' CACAGTGACACACCAACTGGT** 3' | 421 bp | 60°C 1'30" |
| 13A | 5' AGAATACCTGAGAGAGCGGGT 3' | **5' ACGCAAACACACAGATGACTGA** 3' | 265 bp | 60°C 1'30" |
| 14 | 5' CAGTTGAGTGTCAGTGCTATCT 3' | **5' CTGGTGTGAGACAGTATCTCAT** 3' | 492 bp | 60°C 1'30" |
| 15 | 5' ATAAGCATATTCAGAACCAGGCA 3' | 5' CTCCACTTTCACATCTTCTGTG 3' | 340 bp | 60°C 1'30" |
| 16 | 5' TCATCAATTCTGAGGTGCACCA 3' | **5' AGAAACCCGCCCAAACTAAT** 3' | 501 bp | 56°C 30" |
| 17 | 5' **AGCAGATGATATACCACATTGGA** 3' | 5' GTTCATGCCAGTCATTCATCCA 3' | 896 bp | 60°C 30" |
| 18 | 5' AGCAGATGATATACCACATTGGA 3' * | 5' GTTCATGCCAGTCATTCATCCA 3' | 896 bp | 60°C 30" |
| 19 | 5' AGCAGATGATATACCACATTGGA 3' * | 5' GTTCATGCCAGTCATTCATCCA 3' | 896 bp | 60°C 30" |

| | | | | |
|---|---|---|---|---|
| Primers used for sequencing are shown in bold. * Sequence primer for exon 18 is 5' GGTCTTTGCAGGCTATTATA 3' and for exon 19 is 5' GCTCAAATGACATAATTTG | | | | |

Sequence analysis of two Bruck syndrome patients of family PM, showed a G→T nucleotide missense mutation resulting in a Gly→Val amino acid substitution in the sequence GGYENVPT (the mutated Gly is underligned). This mutation was found on both alleles. Both parents were carrier of this missense mutation (see Fig. 3). In one Bruck syndrome patient of family DR a C→T nucleotide missense mutation is seen resulting in a Thr→Ile amino acid substitution in the sequence GGYENVPT. The patient was homozygous for the mutation while both parents and a healthy sister were heterozygous for this mutation (see Fig. 4). The two different point mutations found in family PM and DR were situated in a sequence that shows 100% homology between the different *PLODs* and between different species. This suggests an important role of this region in the function of lysyl hydroxylases in general and that of *PLOD2* in particular. Bruck syndrome is characterized by defective telopeptide lysyl hydroxylase activity [R.A. Bank et al., 1999, Proc. Natl. Acad. Sci. USA, 96: 1054-1058]. The above described mutations show that *PLOD2* encodes telopeptide lysyl hydroxylase.

The knowledge that *PLOD2* encodes for telopeptide lysyl hydroxylase provides the skilled artisan the necessary information for increasing or decreasing the hydroxylysine levels in the telopeptides of collagen by increasing or decreasing the enzyme activity level of telopeptide lysyl hydroxylase in cells during collagen synthesis. The synthesized collagen can subsequently be harvested by standard purification methods for the preparation of collagenous materials (such as collagen matrices as supports for new tissue growth, or collagen-based wound dressings) with increased or decreased turnover times.

### Example 4

### Preparation of collagenous matrices with increased or decreased turnover times

A number of methods has been disclosed to isolate collagen from tissues. This collagen can subsequently be used for the preparation of collagen-based devices. Mostly, skin or tendons are used for the preparation of collagen. The collagen in skin has a very low hydroxyallysine cross-linking. Indeed, collagen in skin has a short half-life. Tendons show various levels of hydroxyallysine cross-links, depending on the anatomical position of the tendon (see e.g. R.A. Bank et al., 1999, Arthr. Rheum., 58: 35-41). The collagen in tendon shows a much higher half-life. Bone shows intermediate levels of hydroxyallysine crosslinks. The amount of hydroxyallysine crosslinks further depends on bone type (cortical or trabecular) and skeletal site (see e.g. L. Knott & A.J. Bailey, 1999, British Poultry Science, 40: 371-379). By mixing the collagen derived from tissues containing low and high hydroxyallysine crosslinks in various ratios, collagen compositions are obtained that can be used for the manufacturing of collagenous matrices showing increased or decreased turnover times.

### Example 5

### Expression of recombinant PLOD2 in cells to enhance lysyl hydroxylation levels in telopeptides

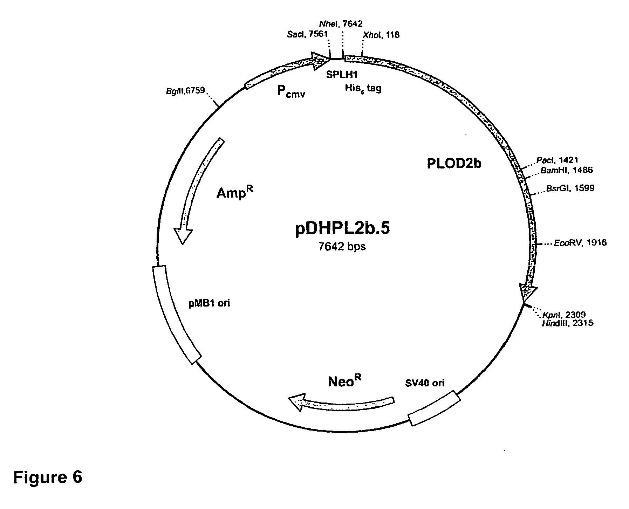

The human *PLOD1* signal peptide sequence followed by a His₆ tag sequence was linked to the cDNA sequence of human *PLOD2B* starting from the likely amino-terminal end of the molecule. Four overlapping oligonucleotides, covering the nucleotide sequence for the *PLOD1* signal peptide and a His₆ tag flanked by *Nhe*I and *Bam*HI restriction sites, were annealed and the protruding 5' ends were filled in by cloned pfu polymerase (Stratagene). The resulting double-stranded, blunt-ended product was ligated to the *Eco*RV site of the pMOSBlue vector (Amersham). The human *PLOD2B* cDNA sequence covering nucleotides 76 to 2283 was cloned into the *Bam*HI/*Kpn*I sites of the construct. Finally, an expression construct (Fig. 6) was created by cloning the *PLOD2B* cDNA sequence with the *PLOD1* signal peptide into the *Nhe*I/*Kpn*I site of pcDNA3.1(-) (invitrogen). A similar construct for splice variant *PLOD2A,* lacking exon 13A, was obtained by replacing a *Pac*I/*Bsr*GI fragment in the *PLOD2B* nucleotide sequence for the same fragment from the PLOD2A sequence. The expression constructs, called pDHPL2b.5 and pDHPL2a.4, were confirmed by sequencing. The recombinant proteins contain a His₆ tag at the N-terminus after signal peptide cleavage. HEK293 cells were cultured in DMEM supplemented with 10% FBS in 5% CO₂ until they reached confluency. For transfection cells were plated in 10 cm² wells at such a density that 70% confluence was reached after 16 hrs of incubation at 37°C. Two hours prior to transfection, fresh medium was added to the cells. The cells were transfected with a total of 1 µg of each plasmid using the lipid-based FuGENE^{™} 6 transfection reagent (Roche Molecular Biochemicals, Indianapolis, Indiana, USA) in a ratio of 1:4 (µg DNA: µl FuGENE). To obtain stable HEK293 clones expressing TLH the cells were diluted 100 times 24 hours after transfection and plated in 10 cm² wells in selective medium containing 700 µg/ml geneticine (Invitrogen). After two weeks culturing in selective medium single clones were picked and screened for TLH expression by Western blotting. Considerable amounts of telopeptide lysyl hydroxylase were observed in both the cytosol and in the culture medium.

The same construct can be used to transfect collagen-producing cells in order to obtain cells that constitutively express telopeptide lysyl hydroxylase encoded by *PLOD2.* By doing so, collagen is secreted showing high levels of hydroxylysine in the telopeptides, being the molecular basis for the generation of collagen matrices with enhanced stability against proteinases. The secreted collagen can be purified by a variety of methods, such as those described by D.K. Furuto & E.J. Miller (1987, Methods Enzymol., 144: 41-61), and used for the preparation of scaffolds.

### Example 6

### Stimulation of endogenous PLOD2 expression in cells to enhance lysy/ hydroxylation levels in telopeptides

Human skin fibroblasts were cultured in 25 cm² flasks in DMEM (Gibco) supplemented with 10% FBS in 5% CO₂. At near confluency the fibroblasts and myofibroblasts were incubated for 50 hours in medium containing 1, 5 or 10 nM human recombinant TGF-β1, -β2 or -β3. As a control, human skin fibroblasts were incubated for 50 hours in medium without the addition of TGF-β. At the end of the incubation period, the cells were washed with PBS and lysed with 600 µl RLT-buffer (RNeasy kit, Qiagen). RNA was isolated following the manufacturers protocol and subsequently reverse transcribed into cDNA (first strand cDNA synthesis kit, Roche Molecular Biochemicals). The levels of *PLOD2B* mRNA in the untreated and TGF-β treated cells were quantified using real-time PCR as described in example 8.

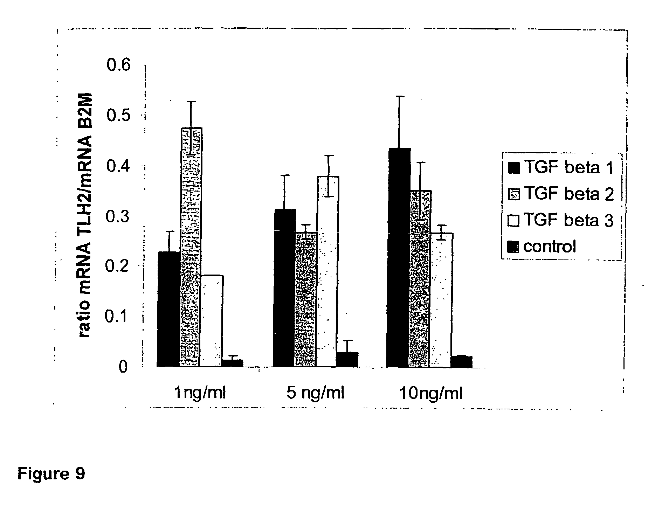

Fig. 9 shows that treatment of fibroblasts with TGF-β1, -β2 or -β3 increases the *PLOD2B* mRNA levels about 20-fold. TGF-β is thus an example of an agent that increases *PLOD2B* mRNA expression and can thus be used to increase cross-linking derived from the hydroxyallysine pathway. Such collagen can be purified and subsequently be used for the preparation of collagen scaffolds showing an increased resistance towards proteinases.

### Example 7

### Inhibition of teloptide lysyl hydroxylase activity levels in cells to decrease lysyl hydroxylation levels in telopeptides

Chondrocytes from the metacarpophalangeal joint of calves (12-14 months old, local slaughterhouse) were isolated by collagenase digestion: cartilage slices were minced and digested overnight at 37°C in 0.14% (w/v) collagenase (Worthington CLS2) in Dulbecco's modified Eagle's medium (DMEM). After filtration of the suspension, cells were washed suspended in 1.2% (w/v) alginate (Keltone LVCR) in 0.9% NaCl at a density of 4 × 10⁶ cells/ml, which was passed dropwise through a 22-gauge needle into 102 mM CaCl₂. After 10 min of polymerization, beads were washed in 0.9% (w/v) NaCl (three times) and finally in complete medium: DMEM-Glutamax (Gibco BRL) supplemented with 100 U/ml of penicillin/streptomycin, 10% (v/v) foetal calf serum and 50 µg/ml ascorbic acid. The cells were cultured at 10 beads per 0.5 ml medium in a humid atmosphere of 5% CO₂ in air at 37°C; the medium was refreshed twice weekly. Minoxidil was dissolved in complete medium and added to the culture after 7 days of preculture under control conditions. Harvested beads were washed with 0.9% NaCl containing 10 mM CaCl₂ and hydrolyzed in 900 µl 6 M HCl at 108°C for 20-24 h. An aliqout of the hydrolysate was subjected to amino acid analysis as decribed by R.A. Bank et al. [1996, Anal. Biochem., 260: 167-176]. Based on hydroxyproline levels, amounts of collagen were calculated, assuming 300 hydroxyproline residues/triple helix. Another aliquot was subjected to crosslink analysis as described by B. Beekman et al. [1997, Exp. Cell Res., 237: 135-141]. Crosslinks were expressed as amount of residues per collagen molecule.

The amount of pyridinolines (HP and LP) in alginate beads cultured in the presence of 1.0 mM or 2.0 mM minoxidil was in mean around 0.15 residues/collagen molecule after a culture period of 20-50 days, whereas the amount of pyridinolines in cultures treated with 0.3 mM minoxidil was in said culture period in mean 0.38 residues/collagen molecule. This shows that increased concentrations of minoxidil reduces the lysyl hydroxylation level of the telopeptides, most likely by means of inhibiting the expression of *PLOD2.*

To substantiate that minoxidil suppresses the expression of *PLOD2,* human skin fibroblasts and myofibroblasts were cultured in 25 cm² flasks in DMEM (Gibco) supplemented with 10% FBS in 5% CO₂. At near confluency the fibroblasts and myofibroblasts were incubated for 41 hours in medium containing 1 mM minoxidil. Minoxidil was dissolved at room temperature in medium with 10% FBS for 8 hours which was filtered prior to use. As a control both cell types were incubated for 41 hours in medium without minoxidil. At the end of the incubation period, the cells were washed with PBS and lysed with 600 µl RLT-buffer (RNeasy kit, Qiagen). RNA was isolated following the manufacturers protocol and subsequently reverse transcribed into cDNA (first strand cDNA synthesis kit, Roche Molecular Biochemicals). The levels of *PLOD2B* mRNA in the untreated and minoxidil-treated cells were quantified using real-time PCR as described in example 8.

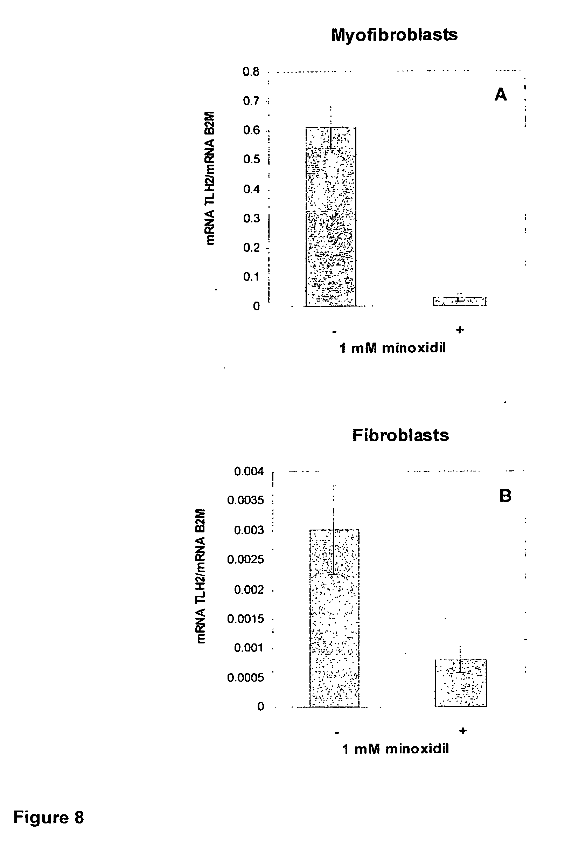

Fig 8 shows that treatment of myofibroblasts with minoxidil reduces the *PLOD2B* mRNA levels about 15-fold. The *PLOD2B* mRNA levels in fibroblasts is much lower than in myofibroblasts (see example 8), but still a 4-fold reduction in mRNA levels can be measured when these cells are cultured in minoxidil containing medium. Minoxidil is thus an example of an inhibitor of *PLOD2B* mRNA expression and can be used to reduce the cross-links derived from the hydroxyallysine pathway. Lysyl oxidase levels were not affected by the minoxidil treatment.

### Example 8

### Inhibition of telopeptide lysyl hydroxylase using antisense oligonucleotides to decrease lysyl hydroxylation levels in telopeptides

The expression of TLH can be downregulated at the post-transcriptional level by degrading the *PLOD2* mRNA using antisense oligonucleotides (AONs). We designed 10 phosphorothioated AONs and tested *in vitro* their potential for duplex formation with the *PLOD2* mRNA and subsequent degradation of the mRNA by RNase H. Non-denatured RNA from 1 x 10⁴ myofibroblasts was incubated for 4 h with 8 µM AON at 37°C in 15 mM Tris-HCl, 2 mM MgCl₂, 50 mM KCl pH 8.0 in a final volume of 25 µl. Degradation of the duplexed mRNA was performed by adding 1 µl 10 mM DTT and 2 u RNase H and incubating the mixture for an additional 20 min at 37 °C. The remaining RNA was reverse transcribed and subjected to PCR with the primers: hPLOD2bRTF (5' TTAAAGGAAAGACACTCCGATCAGAGATGA 3') and hPLOD2aRTR4 (5' TAGCCTTCCAAATTCATGTCTATTAGAAATGTA 3'), which have been chosen such that *PLOD2a* and *PLOD2b* cDNA amplification can be distinguished in a single reaction. Two AONs out of ten were able to decrease the amount of *PLOD2* mRNA to an undetectable level. One recognizes both *PLOD2a* and *PLOD2b* mRNA and has the following sequence: 5' CCCATATTCGGCCCTC 3' (-11-5 nt in the *PLOD2* cDNA sequence) covering the start codon. The second AON with the sequence 5' TTCCCTTTGTAAAGT 3' (1500-1514 nt in the *PLOD2b* cDNA sequence) is specific for *PLOD2b* mRNA and recognizes the extra exon in the PLOD2b sequence. These two AONs are promising for treating for instance myofibroblasts in order to decrease their PLOD2 mRNA levels and as a consequence the amount of HP and LP crosslinks in the extracellular matrix formed by these cells.

### Example 9

### Real-time PCR to measure PLOD2 mRNA levels

RNA, obtained from cultured human skin fibroblasts and cultured human myofibroblast, was isolated using the RNeasy kit (Qiagen). To remove any genomic DNA in the RNA sample, a Dnase treatment was carried out, using RQ1 Rnase-free Dnase (Promega). RNA was then reverse transcripted into cDNA and subjected to real time PCR amplification.

Real time PCR amplification of PLOD2 and β2-microglobulin was performed, using specific primers (PLOD2 forward primer: 5' TTAAAGGAAAGACACTCCGATCAGAGATGA 3'; PLOD2 reverse primer: 5' AATGTTTCCGGAGTAGGGGAGTCTTTTT 3'; β2-microglobulin forward primer: 5' TCTTGTACTACACTGAATTCACCCCCACTGA 3'; β2-microglobulin reverse primer 5' ATCCAAATGCGGCATCTTCAAACCTC 3'), and specific molecular beacons (PLOD2: 5' FAM-cgtgcgCGTGATAAACTGGATCCTGATATGGCTCTT cgcacg-DABCYL 3'; β2-microglobulin: 5' HEX-cgtgcCCTGCCGTGTGAACCATGTGACTTTG gcacg-DABCYL 3'). PLOD2 was amplified in a multiplex PCR together with β2-microglobulin in a total reaction volume of 25µl, containing 1X PCR buffer (Applied Biosystems), 0.4mM of each dNTP, 3.5 mM Mg²⁺, 250nM of each target primer, 100 nM of each primer for β2microglobulin and 1 unit Amplitaq Gold polymerase (Applied Biosystems). PCR was performed in a ABI PRISM^{®} 7700 Sequence Detection System and consisted of a 5 minute interval at 95°C, followed by 40 cycles of 95°C for 30 seconds, 56 °C for 40 seconds and 72 °C for 30 seconds. Data was analysed using sequence detector V1.7 software.

Fig. 5 shows that myofibroblast-like cells show in mean a 40-fold increase of *PLOD2* mRNA levels compared to fibroblasts. Myofibroblasts play a key role in fibrotic processes [C. Badid et al., 2000, Histol. Histopathol., 15: 269-280]. The data indicate that telopeptide lysyl hydroxylase is highly upregulated in fibrotic tissues. This is further corroborated by our observation, that collagen from skin show very low levels of hydroxyallysine cross-links in collagen (0.02 HP residues per triple helix), whereas collagen laid down in tissues containing myofibroblasts show high levels of hydroxyallysine cross-links (0.45 HP residues per triple helix).

As hydroxylation of the telopeptide lysine is controlled by telopeptide lysyl hydroxylase, and as the collagen network containing hydroxyallysine cross-links is more resistant towards proteinases, it is clear that telopeptide lysyl hydroxylase is a key enzyme in fibrosis. The understanding that *PLOD2* encodes telopeptide lysyl hydroxylase provides the skilled artisan with the necessary information for monitoring the onset and/or progression of fibrotic processes by measuring mRNA levels of *PLOD2* and/or protein levels of telopeptide lysyl hydroxylase and/or activity levels of telopeptide lysyl hydroxylase.

### Example 10

### High through-put assay

A peptide containing a hydroxylatable lysine residue, such as biotin-Q-L-S-Y-G-Y-D-E-K-S-T-G-G-I-S-V-P, is dissolved at a concentration of 1 nmol per 0.1 ml PBS. This was added to a mixture of 0.05 ml bovine serum albumin (5 mg albumin Sigma A-7888/ml PBS), 0.05 ml catalase (3.9 ml PBS + 0.1 ml catalase suspesion from Sigma C-100), 0.1 ml 1 mM dithiothreitol, 0.05 ml 20 mM ascorbic acid, 0.05 ml 1 mM ferrous sulfate, 0.1 ml PBS containing the compound to be tested for its inhibitory properties towards telopeptide lysyl hydroxylase, 0.1 ml PBS containing telopeptidase lysyl hydroxylase activity and 0.05 ml α-ketoglutarate. The mixture is incubated for 3 hours at 30°C. An aliquot of the mixture (100 µl) is allowed to bind to the streptavidin coated on 96 wells plate (Pierce) at room temperature for 15 min. The non-bonded fractions were washed away three times with wash buffer (5% Tween-20 in PBS). The bonded peptides are allowed to react with 100 µl of 0.36 µg/ml NalO₄ in 0.025 M phosphate pH 7.0 for 10 min. An additional 3 washing steps are carried out before adding 100 µl of 3.1 µg/ml Texas Red hydrazide in 0.1 M sodium acetate/acetonitril (1:2) pH 4.5. The solution is gently shaken for 2 h at room temperature. After a nine-fold wash step with wash buffer, fluorescence is measured (e.g. with a Cytofluor) at an excitation and emission wave length of 580 nm and 605 nm, respectively. Inhibition of the activity of telopeptide lysyl hydroxylase by the compound in question results in a decrease in the amount of fluorescence, as lysine cannot be converted into an aldehyde in the protocol described above.

Various other hydrazides can be used, such as Lucifer yellow, BODIPYL or Cascade Blue. Instead of biotin, a StrepFlag sequence can be used for binding to the streptavidin. Alternative, other chemistry formats can be chosen, such as a His-tagged peptide in combination with a Ni²⁺ carrier, or a SAMA peptide in combination with maleimide-coated plates.

## Claims

1. A method for producing a collagenous matrix which comprises cross-linked collagen molecules, wherein the resistance of said collagenous matrix against proteolytic degradation is controlled by controlling the ratio of hydroxyl-allysine cross-links to allysine cross-links in the collagenous matrix by controlling the expression of a PLOD2 gene or by controlling the telopeptide lysyl hydroxylase activity of a PLOD2 expression produce.

2. The method of claim 1 wherein the collagen is derived from tissues which show an increased ratio of hydroxyl-allysine cross-links to allysine cross-links and using said collagen to prepare a collagenous matrix with an increased resistance to proteolytic degradation.

3. The method of claim 1 wherein the collagen is derived from tissues which predominantly show allysine cross-links and using said collagen for preparing a collagenous matrix, wherein a collagenous matrix with an increased resistance against proteolytic degradation is obtained by admixing collagen obtained from tissues which shown an increased ratio of hydroxyl-allysine cross-links to allysine cross-links.

4. The method of claim 1 comprising culturing cells producing collagen with telopeptides having an increased ratio of hydroxylysine to lysine residues and using the collagen produced for preparing a collagenous matrix having an increased resistance against proteolytic degradation due to an increased ratio of hydroxyallysine cross-links to allysine cross-links.

5. The method of claim 4 wherein said collagen-producing cells comprise a recombinant, constitutively expressed PLOD2 gene.

6. The method of claim 4 wherein said collagen-producing cells are cultured in the presence of a composition which stimulates the expression of a PLOD2 gene, wherein said stimulation of the expression is achieved by transforming growth factor.

7. The method of claim 1 wherein the collagen is derived from tissues which show a decreased ratio of hydroxyl-allysine cross-links to allysine cross-links and using said collagen to propare a collagenous matrix with a decreased resistance to proteolytic degradation.

8. The method of claim 1 wherein the collagen is derived from tissues which predominantly show hydroxyallysine croas-links and using said collagen for preparing a collagenous matrix, wherein a collagenous matrix with a decreased resistance against proteolytic degradation is obtained by admixing collagen obtained from tissues which shown a decreased ratio of hydroxyl-allysine cross-links to allysine cross-links.

9. The method of claim 1 comprising culturing cells producing collagen with telopeptides having a decreased ratio of hydroxylysine to lysine residues and using the collagen produced for preparing a collagenous matrix having a decreased resistance against proteolytic degradation due to a decreased ratio of hydroxyallysine cross-links to allysine cross-links, wherein said collagen-producing cells are cultured in the presence of a composition which inhibits the activity or production of PLOD-2 encoded telopeptide lysyl hydroxylase, wherein said composition comprises a compound selected from the group of: a silencer of the promoter of the PLOD2 gene, such as minoxidil and minoxidil analogues; an inhibitor of translation of the PLOD2 mRNA, such as anti-sense RNA to the PLOD2 mRNA sequence; an enzyme inhibitor, such as 2-oxoglutarate analogues, chelators of Fe2+, anthracyclines, coumaric acid analogues, a peptide or peptidomimetic containing a non-physiological lysine derivative in a hydroxylatable position wich syncatalytically inactivates said telopeptide lysyl hydroxylase, a hydroxylatable peptide or peptidomimetic that competes with the natural substrate for the active site of said telopeptide lysyl hydroxylase, a non-hydroxylatable peptide or peptidomimetic that competes with the natural substrate for the active site of said telopeptide lysyl hydroxylase, malathion, malaoxon, non-reducing ascorbate analogues; an antibody against the enzyme; a nucleic acid aptamer capable of binding to telopeptide lysyl hydroxylase; or a mutant of telopeptide lysyl hydroxylase.

10. Use of a compound or composition for manufacturing a pharmaceutical preparation for the treatment of a fibrotic condition in a mammal, wherein said compound or composition is capable of reducing the lysyl hydroxylation level of the telopeptides of collagen and thereby in obtaining a collagenous matrix having a decreased ratio of hydroxyallysine cross-links to allysine cross-links, wherein said compound or composition is capable of inhibiting the activity or production of telopeptide lysyl hydroxylase encoded by a PLOD2 gene but not the activity of or production of lysyl oxidase and wherein said compound is selected from the group of: a silencer of the promoter of the PLOD2 gene, such as minoxidil and minoxidil analogues; an inhibitor of translation of the PLOD2 mRNA, such as anti-sense RNA to the PLOD2 mRNA sequence; an enzyme inhibitor, such as 2-oxoglutarate analogues, chelators of Fe2+, anthracyclines, coumaric acid analogues, a peptide or peptidomimetic containing a non-physiological lysine derivative in a hydroxylatable position wich syncatalytically inactivates said telopeptide lysyl hydroxylase, a hydroxylatable peptide or peptidomimetic that competes with the natural substrate for the active site of said telopeptide lysyl hydroxylase, a non-hydroxylatable peptide or peptidomimetic that comperes with the natural substrate for the active site of said telopeptide lysyl hydroxylase, malathion, malaoxon, non-reducing ascorbate analogues; an antibody against the enzyme; a nucleic acid aptamer capable of binding to telopeptide lysyl hydroxylase; or a mutant of telopeptide lysyl hydroxylase.

11. The use of claim 10 wherein said compound or composition comprises a compound that hinds to the subsite or subsites of the active site of said telopeptide lysyl hydroxylase destined for binding of the co-substrate 2-oxoglutarate, thereby replacing the 2-oxoglutarate.

12. The use of claim 10 wherein said compound or composition comprises a compound that cholates the Fe2+ bound in the active site of said telopeptide lysyl hydroxylase, thereby inhibiting the oxygen atom acceptor function of said Fe2+ and/or inhibiting binding of said Fe2+ tot 2-oxoglutatrate.

13. The use of claim 10 wherein said compound or composition comprises an anthracycline or a coumaric acid analogue that syncatalytically inactivates said telopeptide lysyl hydroxylase.

14. The use of claim 10 wherein said compound or composition comprises a peptide or peptidomimetic containing a non-physiological lysine derivative in a hydroxylatable position wich syncatalytically inactivates said telopeptide lysyl hydroxylase.

15. The use of claim 10 wherein said compound or composition comprises a hydroxylatable peptide or peptidomimetic that competes with the natural substrate for the active site of said tolopeptide lysyl hydroxylase.

16. The use of claim 10 wherein said compound or composition comprises a non-hydroxylatable peptide or peptidomimetic that competes with the natural substrate for the active site of said telopeptide lysyl hydroxylase.

17. The use of claim 10 wherein said compound or composition comprises a compound that is a non-reducing ascorbate that binds to the active site of said telopeptide lysyl hydroxylase, thereby replacing ascorbate.

18. The use of claim 10 wherein said compound or composition comprises a peptide or peptidomimetic with a non-hydroxylatable sequence capable of enhancing the uncoupled reaction of said telopeptide lysyl hydroxylase, resulting in an increased level of the inactive self-oxidized form of said telopeptide lysyl hydroxylase.

19. The use of claim 10 wherein said compound or composition comprises a an antibody or aptamer that selectively inhibits the activity of telopeptide lysyl hydroxylase.

20. The use of claim 19 wherein said antibody or aptamer inhibits said telopeptide lysyl hydroxylase by reacting with or near the activo site of said telopeptide lysyl hydroxylase.

21. The use of claim 19 wherein said antibody or aptamer inhibits said telopeptide lysyl hydroxylase by reacting with the telopeptides, thereby inhibiting the reaction of said enzyme by means of steric hindrance.

22. The use of claim 10 wherein said compound or composition comprises a compound that selectively inhibits the transcription of a telopeptide lysyl hydroxylase, wherein said compound is minoxidil or a minoxidil analogue.

23. The use of claim 10 wherein said compound or composition contains an antisense RNA in order to inhibit the translation of mRNA derived from a telopeptide lysyl hydroxylase gene.

24. The use of claim 10 wherein said compound or composition contains a recombinant gene encoding for a mutated telopeptide lysyl hydroxylase that shows no activity towards telopeptides but that is competitive to endogenous telopeptide lysysl hydroxylase with respect to its natural substrate (collagen telopeptides).

25. The use of claim 10 wherein said compound or composition is a hydroxylatable peptide or a sequence encoding such a peptide that is competitive to collagen telopeptides with respect to the reaction that is normally catalyzed by telopeptide lysyl hydroxylase.

26. The use of claim 10 wherein said mammal is a human being.

27. A method for diagnosing and/or monitoring the occurrence or state of a fibrotic process in a mammal comprising taking a sample from said mammal, in vitro analyzing said sample to determine the expression level of a PLOD2 gene and comparing said expression level with a standard.

28. The method of claim 27 wherein the sample is analyzed by Northern blotting or a quantitative or semiquantitative polymerase chain reaction to determine the level of mRNA derived from a PLOD2 gene.

29. The method of claim 27 wherein the sample is analyzed to determine the level of PLOD2-encoded telopeptide lysyl hydroxylase using a PLOD2 specific antibody or aptamer.

30. The method of claim 27 wherein the sample is analyzed to determine the level of enzymatic activity of a PLOD2-encoded telopeptide lysyl hydroxylase using a peptide or polypeptide containing at least one hydroxalatable lysine which is a suitable substrate for PLOD2-encoded telopeptide lysyl hydroxylase.

31. An assay for screening compounds or compositions to determine their effect on telopeptide lysyl hydroxylase activity comprising contacting under enzymatically functional conditions a compound or composition to be tested with a PLOD2-encoded telopoptide lysyl hydroxylase enzyme and a suitable substrate for this enzyme, and determining the level of lysyl hydroxylation of the substrate compared to the level of hydroxylation of the substrate in absence of the compound or composition to be tested.

32. The assay of claim 31 wherein the substrate is a peptide comprising an internal lysine and not having an N-terminal serine or threonine, such as a peptide of the formula Q-L-S-Y-G-Y-D-E-K-S-T-G-G-I-S-V-P, in particular the biotin labeled substrate biotin- Q-L-S-Y-G-Y-D-E-K-S-T-G-G-I-S-V-P.

33. The assay of claim 32 further comprising separating, after said contacting of the compound or composition to be tested with a PLOD2-encoded telopeptide lysyl hydroxylase enzyme and suitable substrate for it, the substrate from the reaction mixture, reacting the substrate successively with an oxidizing agent, such as periodate, which is capable of oxidizing the hydroxyl group of Hyl to an aldehyde moiety, and a hydrazide dye, and measuring the fluorescence from the substrate.

## Patentansprüche

1. Verfahren zur Herstellung einer kollagenhaltigen Matrix, die quervernetzte Kollagenmoleküle umfasst, bei dem die Resistenz der kollagenhaltigen Matrix gegen einen proteolytischen Abbau kontrolliert wird, indem das Verhältnis von Hydroxyallysin-Quervernetzungen zu Allysin-Quervernetzungen in der kollagenhaltigen Matrix kontrolliert wird, indem die Expression eines PLOD2-Gens oder die Telopeptid-Lysylhydroxylase-Aktivität eines PLOD2-Expressionsprodukts kontrolliert wird.

2. Verfahren nach Anspruch 1, bei dem das Kollagen von Geweben erhalten wird, die ein erhöhtes Verhältnis von Hydroxyallysin-Quervernetzungen zu Allysin-Quervernetzungen zeigen, und wobei das Kollagen verwendet wird, um eine kollagenhaltige Matrix mit einer erhöhten Resistenz gegen einen proteolytischen Abbau herzustellen.

3. Verfahren nach Anspruch 1, bei dem das Kollagen von Geweben erhalten wird, die vorwiegend Allysin-Quervernetzungen zeigen, und wobei das Kollagen verwendet wird, um eine kollagenhaltige Matrix herzustellen, wobei eine kollagenhaltige Matrix mit einer erhöhten Resistenz gegen einen proteolytischen Abbau erhalten wird, indem Kollagen zugegeben wird, das von Geweben erhalten wurde, die ein erhöhtes Verhältnis von Hydroxyallysin-Quervernetzungen zu Allysin-Quervernetzungen zeigen.

4. Verfahren nach Anspruch 1, bei dem man Zellen, die Kollagen mit Telopeptiden produzieren, welche ein erhöhtes Verhältnis von Hydroxylysinresten zu Lysinresten aufweisen, kultiviert, und wobei das produzierte Kollagen zur Herstellung einer kollagenhaltigen Matrix verwendet wird, die aufgrund eines erhöhten Verhältnisses von Hydroxyallysin-Quervernetzungen zu Allysin-Quervernetzungen eine erhöhte Resistenz gegen einen proteolytischen Abbau aufweist.

5. Verfahren nach Anspruch 4, bei dem die Kollagen-produzierenden Zellen ein rekombinantes, konstitutiv exprimiertes PLOD2-Gen umfassen.

6. Verfahren nach Anspruch 4, bei dem die Kollagen-produzierenden Zellen in Gegenwart einer Zusammensetzung kultiviert werden, welche die Expression eines PLOD2-Gens stimuliert, wobei die Stimulation der Expression durch einen transformierenden Wachstumsfaktor erreicht wird.

7. Verfahren nach Anspruch 1, bei dem das Kollagen von Geweben erhalten wird, die ein verringertes Verhältnis von Hydroxyallysin-Quervernetzungen zu Allysin-Quervernetzungen zeigen, und wobei das Kollagen verwendet wird, um eine kollagenhaltige Matrix mit einer verringerten Resistenz gegen einen proteolytischen Abbau herzustellen.

8. Verfahren nach Anspruch 1, bei dem das Kollagen aus Geweben erhalten wird, die vorwiegend Hydroxyallysin-Quervernetzungen zeigen, und wobei das Kollagen zur Herstellung einer kollagenhaltigen Matrix verwendet wird, wobei eine kollagenhaltige Matrix mit einer verringerten Resistenz gegen einen proteolytischen Abbau erhalten wird, indem Kollagen zugegeben wird, das von Geweben erhalten wurde, die ein verringertes Verhältnis von Hydroxyallysin-Quervernetzungen zu Allysin-Quervernetzungen zeigen.

9. Verfahren nach Anspruch 1, bei dem man Kollagen-produzierende Zellen mit Telopeptiden kultiviert, die ein verringertes Verhältnis von Hydroxylysinresten zu Lysinresten aufweisen, und wobei das produzierte Kollagen zur Herstellung einer kollagenhaltigen Matrix verwendet wird, die aufgrund eines verringerten Verhältnisses von Hydroxyallysin-Quervernetzungen zu Allysin-Quervernetzungen eine verringerte Resistenz gegen einen proteolytischen Abbau aufweist, wobei die Kollagen-produzierenden Zellen in Gegenwart einer Zusammensetzung kultiviert werden, welche die Aktivität oder die Produktion von PLOD-2-kodierter Telopeptid-Lysylhydroxylase inhibiert, wobei die Zusammensetzung eine Verbindung umfasst die ausgewählt ist aus der folgenden Gruppe: ein Silencer des Promotors des PLOD2-Gens, wie beispielsweise Minoxidil und Minoxidil-Analoga; ein Inhibitor der Translation der PLOD2-mRNA, wie beispielsweise eine Antisense-RNA zu der Sequenz der PLOD2-mRNA; ein Enzyminhibitor, wie beispielsweise 2-Oxoglutarat-Analoga, Chelatoren von Fe²⁺, Anthracycline, Cumarsäure-Analoga, ein Peptid oder Peptidomimetikum, das ein nicht-physiologisches Lysin-Derivat in einer hydroxylierbaren Position aufweist, welches in synkatalytischer Weise die Telopeptid-Lysylhydroxylase inaktiviert, ein hydroxylierbares Peptid oder Peptidomimetikum, das mit dem natürlichen Substrat um das aktive Zentrum der Telopeptid-Lysylhydroxylase konkurriert, ein nicht-hydroxylierbares Peptid oder Peptidomimetikum, das mit dem natürlichen Substrat um das aktive Zentrum der Telopeptid-Lysylhydroxylase konkurriert, Malathion, Malaoxon, nicht-reduzierende Ascorbat-Analoga; ein Antikörper gegen das Enzym; ein Nukleinsäureaptamer, das in der Lage ist, an die Telopeptid-Lysylhydroxylase zu binden; oder eine Mutante der Telopeptid-Lysylhydroxylase.

10. Verwendung einer Verbindung oder Zusammensetzung zur Herstellung einer pharmazeutischen Zubereitung zur Behandlung eines fibrotischen Zustands in einem Säugetier, wobei die Verbindung oder Zusammensetzung in der Lage ist, das Ausmaß der Lysyl-Hydroxylierung des Telopeptids von Kollagen zu verringern, wodurch eine kollagenhaltige Matrix mit einem verringerten Verhältnis von Hydroxyallysin-Quervernetzungen zu Allysin-Quervernetzungen erhalten wird, wobei die Verbindung oder Zusammensetzung in der Lage ist, die Aktivität oder die Produktion von Telopeptid-Lysylhydroxylase, die durch ein PLOD2-Gen kodiert wird, zu inhibieren, jedoch nicht die Aktivität oder die Produktion der Lysyl-Oxydase, und wobei die Verbindung ausgewählt ist aus der folgenden Gruppe: ein Silencer des Promotors des PLOD2-Gens, wie beispielsweise Minoxidil und Minoxidil-Analoga; ein Inhibitor der Translation der PLOD2-mRNA, wie beispielsweise eine Antisense-RNA zu der Sequenz der PLOD2-mRNA; ein Enzyminhibitor, wie beispielsweise 2-Oxoglutarat-Analoga, Chelatoren von Fe²⁺, Anthracycline, Cumarsäure-Analoga, ein Peptid oder Peptidomimetikum, das ein nicht-physiologisches Lysin-Derivat in einer hydroxylierbaren Position aufweist, welches in synkatalytischer Weise die Telopeptid-Lysylhydroxylase inaktiviert, ein hydroxylierbares Peptid oder Peptidomimetikum, das mit dem natürlichen Substrat um das aktive Zentrum der Telopeptid-Lysylhydroxylase konkurriert, ein nicht-hydroxylierbares Peptid oder Peptidomimetikum, das mit dem natürlichen Substrat um das aktive Zentrum der Telopeptid-Lysylhydroxylase konkurriert, Malathion, Malaoxon, nicht-reduzierende Ascorbat-Analoga; ein Antikörper gegen das Enzym; ein Nukleinsäureaptamer, das in der Lage ist, an die Telopeptid-Lysylhydroxylase zu binden; oder eine Mutante der Telopeptid-Lysylhydroxylase.

11. Verwendung nach Anspruch 10, bei der die Verbindung oder Zusammensetzung eine Verbindung umfasst, die an eine Subsite oder an Subsites des aktiven Zentrums der Telopeptid-Lysylhydroxylase bindet, die für die Bindung des Co-Substrats 2-Oxoglutarat bestimmt sind, wodurch 2-Oxoglutarat ersetzt wird.

12. Verwendung nach Anspruch 10, bei der die Verbindung oder Zusammensetzung eine Verbindung umfasst, die das im aktiven Zentrum der Telopeptid-Lysylhydroxylase gebundene Fe²⁺ chelatiert, wodurch die Sauerstoffatom-Akzeptorfunktion des Fe²⁺ inhibiert wird und/oder die Bindung des Fe²⁺ an 2-Oxoglutarat inhibiert wird.

13. Verwendung nach Anspruch 10, bei der die Verbindung oder Zusammensetzung ein Anthracyclin oder ein Cumarsäure-Analog umfasst, das in synkatalytischer Weise die Telopeptid-Lysylhydroxylase inaktiviert.

14. Verwendung nach Anspruch 10, bei der die Verbindung oder Zusammensetzung ein Peptid oder ein Peptidomimetikum umfasst, das ein nicht-physiologisches Lysin-Derivat in einer hydroxylierbaren Position enthält, welches in synkatalytischer Weise die Telopeptid-Lysylhydroxylase inaktiviert.

15. Verwendung nach Anspruch 10, bei der die Verbindung oder Zusammensetzung ein hydroxylierbares Peptid oder ein Peptidomimetikum umfasst, das mit dem natürlichen Substrat um das aktive Zentrum der Telopeptid-Lysylhydroxylase konkurriert.

16. Verwendung nach Anspruch 10, bei der die Verbindung oder Zusammensetzung ein nicht-hydroxylierbares Peptid oder ein Peptidomimetikum umfasst, das mit dem natürlichen Substrat um das aktive Zentrum der Telopeptid-Lysylhydroxylase konkurriert.

17. Verwendung nach Anspruch 10, bei der die Verbindung oder Zusammensetzung eine Verbindung umfasst, die ein nichtreduzierendes Ascorbat ist, welches an das aktive Zentrum der Telopeptid-Lysylhydroxylase bindet, wodurch Ascorbat ersetzt wird.

18. Verwendung nach Anspruch 10, bei der die Verbindung oder Zusammensetzung ein Peptid oder Peptidomimetikum mit einer nicht-hydroxylierbaren Sequenz umfasst, die in der Lage ist, die ungekoppelte Reaktion der Telopeptid-Lysylhydroxylase zu verstärken, was zu einer erhöhten Menge der inaktiven, selbst-oxidierten Form der Telopeptid-Lysylhydroxylase führt.

19. Verwendung nach Anspruch 10, bei der die Verbindung oder Zusammensetzung einen Antikörper oder ein Aptamer umfasst, der/das selektiv die Aktivität der Telopeptid-Lysylhydroxylase inhibiert.

20. Verwendung nach Anspruch 19, bei der der Antikörper oder das Aptamer die Telopeptid-Lysylhydroxylase inhibiert, indem er/es mit dem aktiven Zentrum oder in der Nähe des aktiven Zentrums der Telopeptid-Lysylhydroxylase reagiert.

21. Verwendung nach Anspruch 19, bei der der Antikörper oder das Aptamer die Telopeptid-Lysylhydroxylase inhibiert, indem er/es mit den Telopeptiden reagiert, wodurch die Reaktion des Enzyms durch sterische Behinderung inhibiert wird.

22. Verwendung nach Anspruch 10, bei der die Verbindung oder Zusammensetzung eine Verbindung umfasst, die selektiv die Transkription einer Telopeptid-Lysylhydroxylase inhibiert, wobei die Verbindung Minoxidil oder ein Minoxidil-Analog ist.

23. Verwendung nach Anspruch 10, bei der die Verbindung oder Zusammensetzung eine Antisense-RNA umfasst, um die Translation von mRNA, die von einem Telopeptid-Lysylhydroxylase-Gen abgeleitet ist, zu inhibieren.

24. Verwendung nach Anspruch 10, bei der die Verbindung oder Zusammensetzung ein rekombinantes Gen enthält, das für eine mutierte Telopeptid-Lysylhydroxylase kodiert, welche keine Aktivität gegenüber Telopeptiden zeigt, die jedoch mit endogener Telopeptid-Lysylhydroxylase um deren natürliches Substrat (Kollagen-Telopeptide) konkurriert.

25. Verwendung nach Anspruch 10, bei der die Verbindung oder Zusammensetzung ein hydroxylierbares Peptid oder eine Sequenz ist, die für ein solches Peptid kodiert, das mit Kollagen-Telopeptide um die Reaktion konkurriert, welche normalerweise durch die Telopeptid-Lysylhydroxylase katalysiert wird.

26. Verwendung nach Anspruch 10, bei der das Säugetier ein Mensch ist.

27. Verfahren zur Diagnose und/oder zur Überwachung des Auftretens eines Zustands eines fibrotischen Prozesses in einem Säugetier, bei dem man eine Probe von dem Säugetier nimmt, die Probe in vitro analysiert, um das Expressionsniveau eines PLOD2-Gens zu bestimmen, und das Expressionsniveau mit einem Standard vergleicht.

28. Verfahren nach Anspruch 27, bei dem die Probe durch Northern-Blot oder durch quantitative oder semiquantitative Polymerasekettenreaktion analysiert wird, um die Menge an mRNA, welche von einem PLOD2-Gen abgeleitet ist, zu bestimmen.

29. Verfahren nach Anspruch 27, bei dem die Probe analysiert wird, um die Menge der PLOD2-kodierten Telopeptid-Lysylhydroxylase unter Verwendung eines PLOD2-spezifischen Antikörpers oder eines Aptamers zu bestimmen.

30. Verfahren nach Anspruch 27, bei dem die Probe analysiert wird, um das Ausmaß der enzymatischen Aktivität einer PLOD2-kodierten Telopeptid-Lysylhydroxylase zu bestimmen, wobei ein Peptid oder ein Polypeptid verwendet wird, das mindestens ein hydroxylierbares Lysin enthält, welches ein geeignetes Substrat für die PLOD2-kodierte Telopeptid-Lysylhydroxylase ist.

31. Assay zum Screening nach Verbindungen oder Zusammensetzungen zur Bestimmung ihrer Wirkung auf die Telopeptid-Lysylhydroxylase-Aktivität, bei dem man unter enzymatisch funktionellen Bedingungen eine zu untersuchende Verbindung oder Zusammensetzung mit einem PLOD2-kodierten Telopeptid-Lysylhydroxylase-Enzym und einem geeigneten Substrat für dieses Enzym in Kontakt bringt, und das Ausmaß der Lysyl-Hydroxylierung des Substrats im Vergleich zum Ausmaß der Hydroxylierung des Substrats in Abwesenheit der zu untersuchenden Verbindung oder Zusammensetzung bestimmt.

32. Assay nach Anspruch 31, bei dem das Substrat ein Peptid ist, das ein internes Lysin umfasst, und das kein N-terminales Serin oder Threonin aufweist, wie beispielsweise ein Peptid der Formel Q-L-S-Y-G-Y-D-E-K-S-T-G-G-I-S-V-P, insbesondere das Biotin-markierte Substrat Biotin-Q-L-S-Y-G-Y-D-E-K-S-T-G-G-I-S-V-P.

33. Assay nach Anspruch 32, bei dem man ferner nach dem Inkontaktbringen der zu untersuchenden Verbindung oder Zusammensetzung mit einem PLOD2-kodierten Telopeptid-Lysylhydroxylase-Enzym und einem geeigneten Substrat für dieses das Substrat von der Reaktionsmischung abtrennt, das Substrat nacheinander mit einem Oxidationsmittel umsetzt, wie beispielsweise Periodat, das in der Lage ist, die Hydroxylgruppe von Hyl in einen Aldehydrest zu oxidieren, sowie mit ein Hydrazidfarbstoff, und wobei die Fluoreszenz des Substrats gemessen wird.

## Revendications

1. Procédé de production d'une matrice collagène comprenant des molécules de collagène réticulées, dans lequel la résistance de ladite matrice collagène à la dégradation protéolytique est contrôlée en régulant le ratio des ponts hydroxyallysine sur les ponts allysine dans la matrice collagène par le contrôle de l'expression d'un gène PLOD2 ou par le contrôle de l'activité de télopeptide lysyle hydroxylase d'un produit d'expression du gène PLOD2.

2. Procédé selon la revendication 1, dans lequel le collagène provient de tissus présentant un ratio des ponts hydroxyallysine sur les ponts allysine accru et utilisant ledit collagène pour préparer une matrice collagène ayant une résistance accrue à la dégradation protéolytique.

3. Procédé selon la revendication 1, dans lequel le collagène provient de tissus présentant de manière prédominante des ponts allysine et utilisant ledit collagène pour préparer une matrice collagène, la matrice collagène ayant une résistance accrue à la dégradation protéolytique étant obtenue par l'incorporation de collagène dérivé de tissus présentant un ratio des ponts hydroxyallysine sur les ponts allysine accru.

4. Procédé selon la revendication 1, comprenant la mise en culture de cellules productrices de collagène avec des télopeptides présentant un ratio des résidus hydroxylysine sur les résidus lysine accru et l'utilisation du collagène produit pour préparer une matrice collagène présentant une résistance accrue à la dégradation protéolytique en raison d'un ratio des ponts hydroxyallysine sur les ponts allysine accru.

5. Procédé selon la revendication 4, dans lequel lesdites cellules productrices de collagène comprennent un gène recombinant PLOD2 exprimé constitutivement.

6. Procédé selon la revendication 4, dans lequel lesdites cellules productrices de collagène sont mises en culture en présence d'une composition qui stimule l'expression d'un gène PLOD2, ladite stimulation de l'expression étant obtenue par un facteur de croissance transformant.

7. Procédé selon la revendication 1, dans lequel le collagène provient de tissus présentant un ratio ponts hydroxyallysine sur les ponts allysine réduit et utilisant ledit collagène pour préparer une matrice collagène ayant une résistance réduite à la dégradation protéolytique.

8. Procédé selon la revendication 1, dans lequel le collagène provient de tissus présentant de manière prédominante des ponts hydroxyallysine et utilisant ledit collagène pour préparer une matrice collagène, la matrice collagène ayant une résistance réduite à la dégradation protéolytique étant obtenue par l'incorporation de collagène dérivé de tissus présentant un ratio des ponts hydroxyallysine sur les ponts allysine réduit.

9. Procédé selon la revendication 1, comprenant la mise en culture de cellules productrices de collagène avec des télopeptides présentant un ratio des résidus hydroxylysine sur les résidus lysine réduit et utilisant le collagène produit pour préparer une matrice collagène présentant une résistance réduite à la dégradation protéolytique en raison d'un ratio des ponts hydroxyallysine sur les ponts allysine réduit, lesdites cellules productrices de collagène étant mises en culture en présence d'une composition qui inhibe l'activité ou la production de télopeptide lysyle hydroxylase codée par le PLOD2, ladite composition comprenant un composé sélectionné dans le groupe formé par : un silenceur du promoteur du gène PLOD2 tel que le minoxidil et ses analogues ; un inhibiteur de la traduction de l'ARNm du PLOD2 tel que l'ARN antisens de la séquence d'ARNm du PLOD2 ; un inhibiteur d'enzyme tel que les analogues du 2-oxoglutarate, les chélatants du Fe²⁺, les anthracyclines, les analogues de l'acide coumarique, un peptide ou peptidomimétique contenant un dérivé de lysine non physiologique dans une position hydroxylable qui inactive de manière syncatalytique ladite télopeptide lysyle hydroxylase, un peptide ou peptidomimétique hydroxylable qui entre en compétition avec le substrat naturel pour le site actif de ladite télopeptide lysyle hydroxylase, un peptide ou peptidomimétique non hydroxylable qui entre en compétition avec le substrat naturel pour le site actif de ladite télopeptide lysyle hydroxylase, le malathion, le malaoxon, les analogues d'ascorbates non réducteurs ; un anticorps dirigé contre l'enzyme ; un aptamère d'acide nucléique capable de se lier à la télopeptide lysyle hydroxylase ; ou un mutant de la télopeptide lysyle hydroxylase.

10. Utilisation d'un composé ou d'une composition pour produire une préparation pharmaceutique destinée au traitement d'un état fibrotique chez un mammifère, ledit composé ou ladite composition étant capable de réduire le niveau de lysylhydroxylation des télopeptides du collagène et permettant ainsi d'obtenir une matrice collagène présentant un ratio des ponts hydroxyallysine sur les ponts allysine réduit, ledit composé ou ladite composition étant capable d'inhiber l'activité ou la production de la télopeptide lysyle hydroxylase codée par un gène PLOD2 mais pas l'activité ou la production de la lysyle oxydase, ledit composé étant sélectionné dans le groupe formé par : un silenceur du promoteur du gène PLOD2 tel que le minoxidil et ses analogues ; un inhibiteur de la traduction de l'ARNm du PLOD2 tel que l'ARN antisens de la séquence d'ARNm du PLOD2 ; un inhibiteur d'enzyme tel que les analogues du 2-oxoglutarate, les chélatants du Fe²⁺, les anthracyclines, les analogues de l'acide coumarique, un peptide ou peptidomimétique contenant un dérivé de lysine non physiologique dans une position hydroxylable qui inactive de manière syncatalytique ladite télopeptide lysyle hydroxylase, un peptide ou peptidomimétique hydroxylable qui entre en compétition avec le substrat naturel pour le site actif de ladite télopeptide lysyle hydroxylase, un peptide ou peptidomimétique non hydroxylable qui entre en compétition avec le substrat naturel pour le site actif de ladite télopeptide lysyle hydroxylase, le malathion, le malaoxon, les analogues d'ascorbates non réducteurs ; un anticorps dirigé contre l'enzyme ; un aptamère d'acide nucléique capable de se lier à la télopeptide lysyle hydroxylase ; ou un mutant de la télopeptide lysyle hydroxylase.

11. Utilisation selon la revendication 10, dans laquelle ledit composé ou ladite composition comprend un composé qui se lie au(x) sous-site(s) du site actif de ladite télopeptide lysyle hydroxylase destiné(s) à la liaison du co-substrat 2-oxoglutarate, remplaçant ainsi le 2-oxoglutarate.

12. Utilisation selon la revendication 10, dans laquelle ledit composé ou ladite composition comprend un composé qui chélate le Fe²⁺ lié au site actif de ladite télopeptide lysyle hydroxylase, inhibant ainsi la fonction d'accepteur d'atomes d'oxygène dudit Fe²⁺ et/ou inhibant la liaison dudit Fe²⁺ avec le 2-oxpglutarate.

13. Utilisation selon la revendication 10, dans laquelle ledit composé ou ladite composition comprend une anthracycline ou un analogue de l'acide coumarique qui inactive syncatalytiquement ladite télopeptide lysyle hydroxylase.

14. Utilisation selon la revendication 10, dans laquelle ledit composé ou ladite composition comprend un peptide ou un peptidomimétique contenant un dérivé de lysine non physiologique dans une position hydroxylable qui inactive syncatalytiquement ladite télopeptide lysyle hydroxylase.

15. Utilisation selon la revendication 10, dans laquelle ledit composé ou ladite composition comprend un peptide ou un peptidomimétique hydroxylable qui entre en compétition avec le substrat naturel pour le site actif de ladite télopeptide lysyle hydroxylase.

16. Utilisation selon la revendication 10, dans laquelle ledit composé ou ladite composition comprend un peptide ou un peptidomimétique non hydroxylable qui entre en compétition avec le substrat naturel pour le site actif de ladite télopeptide lysyle hydroxylase.

17. Utilisation selon la revendication 10, dans laquelle ledit composé ou ladite composition comprend un composé qui est un ascorbate non réducteur qui se lie au site actif de ladite télopeptide lysyle hydroxylase, remplaçant ainsi l'ascorbate.

18. Utilisation selon la revendication 10, dans laquelle ledit composé ou ladite composition comprend un peptide ou un peptidomimétique présentant une séquence non hydroxylable capable de renforcer la réaction non couplée de ladite télopeptide lysyle hydroxylase, ce qui a pour effet d'augmenter le niveau de la forme auto-oxydée inactive de ladite télopeptide lysyle hydroxylase.

19. Utilisation selon la revendication 10, dans laquelle ledit composé ou ladite composition comprend un anticorps ou un aptamère qui inhibe sélectivement l'activité de la télopeptide lysyle hydroxylase.

20. Utilisation selon la revendication 19, dans laquelle ledit anticorps ou ledit aptamère inhibe ladite télopeptide lysyle hydroxylase en réagissant avec le site actif de ladite télopeptide lysyle hydroxylase ou au voisinage de ce site.

21. Utilisation selon la revendication 19, dans laquelle ledit anticorps ou ledit aptamère inhibe ladite télopeptide lysyle hydroxylase en réagissant avec les télopeptides, inhibant ainsi la réaction de ladite enzyme par un empêchement stérique.

22. Utilisation selon la revendication 10, dans laquelle ledit composé ou ladite composition comprend un composé qui inhibe sélectivement la transcription d'une télopeptide lysyle hydroxylase, ledit composé étant le minoxidil ou un analogue du minoxidil.

23. Utilisation selon la revendication 10, dans laquelle ledit composé ou ladite composition contient un ARN antisens afin d'inhiber la traduction de l'ARNm dérivé d'un gène codant pour la télopeptide lysyle hydroxylase.

24. Utilisation selon la revendication 10, dans laquelle ledit composé ou ladite composition contient un gène recombinant codant pour une télopeptide lysyle hydroxylase mutée qui ne présente aucune activité vis-à-vis des télopeptides mais qui entre en compétition avec la télopeptide lysyle hydroxylase endogène pour son substrat naturel (télopeptides du collagène).

25. Utilisation selon la revendication 10, dans laquelle ledit composé ou ladite composition est un peptide hydroxylable ou une séquence codant pour un tel peptide qui entre en compétition avec les télopeptides du collagène pour la réaction qui est normalement catalysée par la télopeptide lysyle hydroxylase.

26. Utilisation selon la revendication 10, dans laquelle ledit mammifère est un être humain.

27. Procédé de diagnostic et/ou de surveillance de l'apparition ou de l'état d'un processus de fibrose chez un mammifère, comprenant le prélèvement d'un échantillon chez ledit mammifère, l'analyse *in vitro* dudit échantillon afin de déterminer le niveau d'expression du gène PLOD2 et la comparaison dudit niveau d'expression à un étalon.

28. Procédé selon la revendication 27, dans lequel l'échantillon est analysé par buvardage de Northem ou par une amplification en chaîne par polymérase quantitative ou semi-quantitative afin de déterminer le niveau d'ARNm transcrit à partir d'un gène PLOD2.

29. Procédé selon la revendication 27, dans lequel l'échantillon est analysé pour déterminer le niveau de télopeptide lysyle hydroxylase codée par le PLOD2 en utilisant un anticorps ou un aptamère spécifique au PLOD2.

30. Procédé selon la revendication 27, dans lequel l'échantillon est analysé pour déterminer le niveau d'activité enzymatique d'une télopeptide lysyle hydroxylase codée par le PLOD2 en utilisant un peptide ou polypeptide qui contient au moins une lysine hydroxylable qui est un substrat adéquat pour la télopeptide lysyle hydroxylase codée par le PLOD2.

31. Essai de criblage de composés ou de compositions afin de déterminer leur effet sur l'activité de la télopeptide lysyle hydroxylase, comprenant, dans des conditions enzymatiquement fonctionnelles, la mise en contact d'un composé ou d'une composition à tester avec une enzyme télopeptide lysyle hydroxylase codée par le PLOD2 et avec un substrat adéquat pour cette enzyme, et la détermination du niveau de lysylhydroxylation du substrat comparé au niveau d'hydroxylation du substrat en l'absence du composé ou de la composition à tester.

32. Essai selon la revendication 31, dans lequel le substrat est un peptide comprenant une lysine interne et dépourvu de sérine ou thréonine N-terminale, tel qu'un peptide de formule Q-L-S-Y-G-Y-D-E-K-S-T-G-G-I-S-V-P, notamment le substrat biotinylé biotine-Q-L-S-Y-G-Y-D-E-K-S-T-G-G-I-S-V-P

33. Essai selon la revendication 32, comprenant en plus, après ladite mise en contact du composé ou de la composition à tester avec une enzyme télopeptide lysyle hydroxylase codée par le PLOD2 et avec un substrat adéquat pour ladite enzyme, la séparation du substrat d'avec le mélange réactionnel, suivie de la réaction du substrat avec un agent oxydant, tel que le periodate, capable d'oxyder le groupe hydroxyle de l'hydroxylysine (Hyl) pour donner un fragment aldéhyde, et avec un colorant hydrazide, puis de la mesure de la fluorescence du substrat.
